# EUROPEAN PATENT SPECIFICATION

(11) **EP 3 200 704 B1**
(45) Date of publication and mention of the grant of the patent: **20.05.2020**
(21) Application number: 15859643.7
(22) Date of filing: 30.09.2015
(51) Int. Cl.: A61B 17/17

(54) **INSTRUMENTS FOR MINIMALLY INVASIVE SURGICAL PROCEDURES**
INSTRUMENTE FÜR MINIMALINVASIVE CHIRURGISCHE EINGRIFFE
INSTRUMENTS POUR INTERVENTIONS CHIRURGICALES MINI-INVASIVES

(30) Priority: 30.09.2014 US 201462057415 P; 05.12.2014 US 201462088160 P; 05.12.2014 US 201462088103 P; 06.02.2015 US 201562113163 P; 26.02.2015 US 201562121108 P; 25.09.2015 US 201562232654 P
(43) Date of publication of application: 09.08.2017
(73) Proprietor: Massachusetts Institute of Technology, Cambridge, MA 02139 (US); The General Hospital Corporation, Boston, MA 02114 (US)
(72) Inventor: JOHNSON, Matthew, Roy, Lexington, MA 02420 (US); HILL, Westin, Michael, Durham, NC 27701 (US); CODD, Patrick, James, Boston, MA 02114 (US)
(74) Representative: McGlashan, Graham Stewart
(86) International application number: PCT/US2015/053358
(87) International publication number: WO 2016/076964

(56) References cited:
- WO-A2-2007/059233
- US-A1- 2002 068 868
- US-A1- 2005 020 901
- US-A1- 2008 091 170
- US-A1- 2008 195 081
- US-A1- 2011 071 544
- US-A1- 2011 092 810
- US-A1- 2013 018 303
- US-A1- 2014 276 925

## Description

### TECHNICAL FIELD

The present disclosure generally relates to various surgical instruments and their applications in minimally invasive surgeries.

### BACKGROUND

There remains a need for improved devices, systems, and methods for minimally invasive surgical procedures.

US2005020901 describes using an endoscopic device having an automatically controllable proximal portion and a selectively steerable distal portion, wherein the device generally may be advanced into the body through an opening. The distal portion is selectively steered to assume a selected curve along a desired path within the body which avoids contact with tissue while the proximal portion is automatically controlled to assume the selected curve of the distal portion. The endoscopic device can then be used for accessing various regions of the body which are typically difficult to access and treat through conventional surgical techniques because the device is unconstrained by "straight-line" requirements. Various applications can include accessing regions of the brain, thoracic cavity, including regions within the heart, peritoneal cavity, etc., which are difficult to reach using conventional surgical procedures.

WO2007059233 describes a surgical needle, or active cannula, that is capable of following a complex path through cavities and tissue within a patient's anatomy. The needle has a plurality of overlapping flexible tubes, each of which has a pre-formed curvature and a pre-determined flexibility. Each of the plurality of flexible tubes is selected based on their respective pre-formed curvature and flexibility so that a given overlap configuration causes the combination of overlapping flexible tubes to form a predetermined shape that substantially matches a desired path through the anatomy. By individually controlling the translation and angular orientation of each of the flexible tubes, the surgical needle may be guided through the anatomy according to the desired path.

### SUMMARY

Aspects of the present invention are defined in the independent claims. Preferred features are defined in the dependent claims.

A variety of tools and techniques are provided to support removal of ligamentum flavum in a spinal canal and other, similar tissue removal procedures.

A method may include providing a model of a surgical canal of a patient, where the surgical canal includes a surgical entry point, a supporting tissue region, a soft tissue region, and a surgical target, and the surgical canal has a curvature. The method may further include forming a surgical tool having a distal tip into a compound curve, where the compound curve includes at least a first bend positioned to provide a point of contact between the surgical tool and the supporting tissue region of the surgical canal when the distal tip is positioned for a procedure on the surgical target, thereby providing mechanical support for a position of the distal tip, and the compound curve includes a second bend positioned to direct the distal tip toward the surgical target.

Implementations may have one or more of the following features. The model may include a two-dimensional image of the surgical canal. The two-dimensional image may be a cross section of a spinal canal of a patient. The surgical tool may include at least one of an endoscope and a cannula for the endoscope. The method may further include positioning a balloon at a location between the surgical tool and the soft tissue region, inflating the balloon to provide a soft barrier between the surgical tool and the soft tissue region, steering the distal tip of the surgical tool, steering the distal tip toward the surgical target, steering the distal tip away from the soft tissue region, steering the distal tip with a wire mechanically coupled through the surgical tool from a control end to the distal tip or by applying an external magnetic field to impart a force to move one or more magnets coupled to the surgical tool, and/or steering the distal tip by rotating or translating one or more concentrically nested tubes within the surgical tool having different curvatures or stiffnesses.

In an aspect, a device includes a surgical tool having a body, a control end, and a distal end, where the body is shaped and sized for insertion past a sacral hiatus and into a spinal canal of a patient, the control end is positioned outside the patient when the surgical tool is positioned for use, and the distal end is positioned inside the spinal canal when the surgical tool is positioned for use. The device may further include a compound curve in the body of the surgical tool, where the compound curve includes at least a first bend positioned to provide a point of contact between the surgical tool and a supporting tissue region of the spinal canal when a distal tip of the distal end is positioned for a procedure on a surgical target within the spinal canal, thereby providing mechanical support for a position of the distal tip, and the compound curve includes a second bend positioned to direct the distal tip toward the surgical target.

Implementations may have one or more of the following features. The first bend may have a predetermined stiffness selected to deform to a native curvature of the spinal canal in order to retain adequate mating contact between the body and a wall of the spinal canal in a region between a first apex of the first bend and a second apex of the second bend to retain the distal end in a fixed position relative to the surgical target. The predetermined stiffness may retain the second apex in contact with the wall of the spinal canal. The second bend may direct the distal end toward the surgical target when the surgical tool is placed for use. The surgical tool may include an endoscope or a cannula for an endoscope. The device may further include at least one of a magnet coupled to the body near the distal end, a control wire coupled between the control end and the distal end to control a positon of the distal end relative to the body, one or more concentrically nested and curved tubes within the body operable to control a curvature of the second bend, and one or more balloons coupled to an exterior of the body and inflatable to move the body within the spinal canal. The body may have an outside diameter not greater than 3 mm. The device may further include a surgical laser and an optical fiber positioned to transmit optical energy from the surgical laser through the body to the distal tip, and/or an electrosurgical radio frequency device coupled to the distal end and operable for radio frequency ablation of the surgical target.

In another aspect, a device includes a first tube having a proximal end for manipulation during a surgical procedure, a distal end for insertion into a surgical site, a hollow core, and an exit from the hollow core at the distal end. The device may also include a plurality of flaps coupled to the distal end of the first tube, a second tube slidably disposed within and concentric to the first tube, and a controller operably configured to move the plurality of flaps relative to the first tube between an undeployed state where the plurality of flaps are contained within a cross section normal to a longitudinal axis of the first tube and a deployed state where the plurality of flaps are at least in part disposed outside of the cross section to provide a mechanical separation between the distal end of the first tube and tissue surrounding a surgical site.

Implementations may have one or more of the following features. The second tube may include a plurality of elements that each mechanically mate with a corresponding element on one of the plurality of flaps, such that sliding the second tube in a first direction relative to the first tube causes the plurality of elements to engage the plurality of flaps and move each of the plurality of flaps from the undeployed state to the deployed state. Sliding the second tube in a second direction opposite to the first direction may disengage the plurality of elements from the plurality of flaps and an interior surface of the first tube urges the plurality of flaps into the undeployed state. Each of the plurality of flaps may be spring-biased toward the deployed state and the first tube retains the plurality of flaps in the undeployed state when the second tube is in an undeployed position. The plurality of flaps may be integrally formed with the first tube, and/or bear a smoothly contoured shape free of sharp edges and corners. The device may further include a laser surgical tool at the distal end of the first tube and coupled to a second controller at the proximal end of the first tube, and/or an electrosurgical radio frequency device at the distal end of the first tube and coupled to a second controller at the proximal end of the first tube. The first tube may have a second hollow core parallel to the hollow core for one or more supplemental tools. The device may further include an imaging element at the distal end, wherein the one or more supplemental tools includes saline wash sourced through the second hollow core and directed at the imaging element. The imaging element may include at least one of a digital camera and an optical fiber for optically transmitting an image at the distal end to the proximal end. The device may further include an illumination source at the distal end coupled to a power and control source at the proximal end of the first tube, a mechanical locking mechanism at the distal end configured to removably and replaceably receive a supplemental surgical tool, at least one of a piercing tool, a cutting tool, a hooking tool, and a grasping tool coupled to the mechanical locking mechanism, a protective shield at the distal end of the first tube formed of a material selected to protect a surgical site from laser energy provided by a surgical laser operating at the distal end of the first tube, at least one steering wire coupling the protective shield to a second controller on the proximal end of the first tube to facilitate positional control of the protective shield from the proximal end during a surgical procedure, one or more concentrically nested and curved tubes within the first tube operable to control a curvature of the first tube, at least one of a magnet coupled to the device near the distal end, a control wire coupled between the proximal end and the distal end to control a positon of the distal end relative to the proximal end, and one or more balloons coupled to an exterior of the first tube and inflatable to move the first tube within a surgical cavity, and/or an endoscope positioned within the first tube.

In yet another aspect, a device includes a first tube having a proximal end for manipulation during a surgical procedure, a distal end for insertion into a surgical site, a hollow core, and an exit from the hollow core at the distal end. The device may also include a second tube slidably disposed and concentric with the first tube, the second tube having a distal end, a plurality of flaps coupled to the distal end of the second tube, and a controller operably configured to move the plurality of flaps relative to the second tube between an undeployed state where the plurality of flaps are contained within a cross section normal to a longitudinal axis of the second tube and a deployed state where the plurality of flaps are at least in part disposed outside of the cross section to provide a mechanical separation between the distal end of the second tube and tissue surrounding a surgical site.

In another aspect, a device includes a flexible endoscopic device with a body having a distal end for insertion into a surgical site and a proximal end for operator control, an outer sheath disposed on the distal end of the body, an inner sheath disposed inside of and concentric with the outer sheath on the distal end of the body, and a void disposed between the outer sheath and the inner sheath on the distal end of the body, where the void is in fluid connection with the proximal end of the body whereby a suction created at the proximal end of the body creates a reduced pressure in the void between the outer sheath and the inner sheath on the distal end of the body relative to an ambient pressure. The device may also include a cutting tool rotatably disposed inside of and concentric with the inner sheath, where the cutting tool is configured to rotate relative to the inner sheath, where rotation of the cutting tool in a first direction about an axis extends a sharp edge of the cutting tool beyond a leading edge of the inner sheath, and where rotation of the cutting tool in a second direction about the axis retracts the sharp edge of the cutting tool such that it is fully sheathed by the leading edge of the inner sheath. The device may further include a controller at the proximal end including a first control to create suction at the proximal end of the body and a second control to rotate the cutting tool relative to the inner sheath.

Implementations may have one or more of the following features. The device may further include a vacuum source at the proximal end of the body coupled in fluid communication with the void. The vacuum source may be configured to apply a variable vacuum force under control of the controller. The controller may be configured to infer a vacuum sealed engagement of the distal end with a surface based upon an amount of pressure created in the void by the vacuum source. The controller may be configured to prevent operation of the cutting tool when no vacuum engagement of the distal end to a surface is detected. The body may have a first hollow core for a surgical device. The device may further include a laser surgical tool at the distal end of the body and coupled to the controller at the proximal end through the first hollow core, an electrosurgical radio frequency device at the distal end of the body and coupled to the controller at the proximal end through the first hollow core, a second hollow core in the body parallel to the first hollow core for one or more supplemental tools, and/or an imaging element at the distal end, wherein the one or more supplemental tools includes saline wash sourced through the second hollow core and directed at the imaging element. The imaging element may include a digital camera at the distal end. The imaging element may include an optical fiber for optically transmitting an image at the distal end to the proximal end. The device may further include an illumination source at the distal end coupled to a power and control source at the proximal end, a mechanical locking mechanism at the distal end configured to removably and replaceably receive a supplemental surgical tool, at least one of a piercing tool, a hooking tool, a cutting tool, and a grasping tool coupled to the mechanical locking mechanism, a protective shield at the distal end of the body formed of a material selected to protect the surgical site from laser energy provided by a surgical laser operating at the distal end of the body, at least one steering wire coupling the protective shield to a second controller on the proximal end of the body to facilitate positional control of the protective shield from the proximal end during a surgical procedure, one or more concentrically nested and curved tubes within the flexible endoscopic device to control a curvature of a tool included therein, at least one of a magnet coupled to the device near the distal end, a control wire coupled between the proximal end and the distal end to control a positon of the distal end relative to the proximal end, and one or more balloons coupled to an exterior of the body and inflatable to move the body within a surgical cavity, and/or an endoscope positioned within the body.

In an aspect, an endoscopic device for surgical removal of ligamentum flavum includes a body having a first end for insertion into a patient, a second end for controlling a surgical procedure, and a hollow core within the body between the first end and the second end, where the body is shaped and sized for insertion into a human spinal canal. The endoscopic device may also include a camera on the first end, where the camera has a field of view projecting from the first end and the camera coupled through the hollow core to a display on the second end. The endoscopic device may further include a laser coupled to the second end and operable to provide coherent light at an infrared wavelength selected to ablate ligamentum flavum through fracturing of extracellular matrix due to thermal heating of water preferentially over direct photodecomposition of extracellular matrix, and a fiber optic having a laser end coupled to the laser to direct an output of the laser through the hollow core to the first end of the body of the endoscopic device and out an output end toward a target within the field of view of the camera, the fiber optic including an optical element that focuses a beam exiting the output end into a fluence spatial pattern having steep sides to reduce heating of peripheral non-ablated tissue of a target surface exposed to the beam. The endoscopic device may also include a controller coupled to the laser and programmed to deliver a sequence of pulses from the laser through the fiber optic toward the target with a sufficient fluence for bulk tissue removal of ligamentum flavum.

Implementations may have one or more of the following features. The optical element may include a lens on the output end of the fiber optic that focuses the beam into a diffraction limited beam, and or a low Numerical Aperture of the fiber optic selected to reduce divergence of the beam at the output end of the fiber optic. The fluence spatial pattern may be a substantially top-hat pattern, and/or have side lobes at least 30 dB below a peak of the fluence spatial pattern. The laser may include a Ho:YAG laser, and/or outputs light at a wavelength of about 2080 nanometers. The fiber optic may have a low hydroxyl content not exceeding two parts per million. The endoscopic device may further include a Q-switch, where the controller is configured to deliver pulses with a length of not more than 260 nanoseconds (full width half maximum). The controller may be configured to deliver pulses with a length of not more than 140 microseconds (full width half maximum). The endoscopic device may further include a saline source coupled to a saline output at the first end of the body, where the saline output is directed toward the target at the output end of the fiber optic. The endoscopic device may further include a steering mechanism to control a direction of the output end relative to the body of the endoscopic device.

In another aspect, an endoscopic device for surgical removal of ligamentum flavum includes a body having a first end for insertion into a patient, a second end for controlling a surgical procedure, and a hollow core within the body between the first end and the second end, the body shaped and sized for insertion into a human spinal canal. The endoscopic device may also include a camera on the first end, the camera having a field of view projecting from the first end and the camera coupled through the hollow core to a display on the second end. The endoscopic device may further include a laser coupled to the second end and operable to provide coherent light at an infrared wavelength selected to ablate ligamentum flavum through fracturing of extracellular matrix due to thermal heating of water preferentially over direct photodecomposition of extracellular matrix, a Q-switch within an optical resonator of the laser, the Q-switch operable to produce a pulsed laser output from the laser, an optical fiber having a laser end coupled to the laser to direct an output of the laser through the hollow core to the first end of the body of the endoscopic device and out an output end toward a target within the field of view of the camera, and a controller coupled to the laser and the Q-switch and programmed to deliver a sequence of Q-switched pulses from the laser through the optical fiber toward the target with a sufficient fluence for bulk tissue removal of ligamentum flavum and with a pulse length that is sufficiently short to prevent regions of reduced fluence in a beam exiting from the output end from causing non-ablative heating damage to regions of tissue around a periphery of the target.

Implementations may have one or more of the following features. The Q-switch may include an acousto-optic attenuator. The laser may include a Ho:YAG laser. The optical fiber may have a low hydroxyl content not exceeding two parts per million. The controller may be configured to deliver pulses with a length of not more than 20 nanoseconds (full width half maximum), configured to deliver pulses with a length of not more than 1 picosecond (full width half maximum), and/or configured to deliver pulses with a length selected for athermal ablation of ligamentum flavum. The endoscopic device may further include a steering mechanism to control a direction of the output end relative to the body of the endoscopic device.

In yet another aspect, an endoscopic device for surgical removal of ligamentum flavum includes a body having a first end for insertion into a patient, a second end for controlling a surgical procedure, and a hollow core within the body between the first end and the second end, the body shaped and sized for insertion into a human spinal canal. The endoscopic device may also include a camera on the first end, the camera having a field of view projecting from the first end and the camera coupled through the hollow core to a display on the second end, a laser coupled to the second end and operable to provide coherent light at an ultraviolet wavelength selected to ablate ligamentum flavum through direct photodecomposition of extracellular matrix preferentially over fracturing of extracellular matrix due to thermal heating of water, a Q-switch within an optical resonator of the laser, the Q-switch operable to produce a pulsed laser output from the laser, an optical fiber having a laser end coupled to the laser to direct an output of the laser through the hollow core to the first end of the body of the endoscopic device and out an output end toward a target within the field of view of the camera, and a controller coupled to the laser and the Q-switch and programmed to deliver a sequence of Q-switched pulses from the laser through the optical fiber toward the target with a sufficient fluence for bulk tissue removal of ligamentum flavum.

Implementations may have one or more of the following features. The laser may include a frequency quadrupled Nd:YAG laser, output light at a wavelength of about 266 nanometers, and/or output light at a wavelength less than 300 nanometers. The Q-switch may include an acousto-optic attenuator. The optical fiber may have a high hydroxyl content not less than 600 parts per million. The controller may be configured to deliver pulses with a length of not more than 20 nanoseconds (full width half maximum), configured to deliver pulses with a length of not more than 5 nanoseconds (full width half maximum), configured to deliver pulses with a length of not more than 1 nanosecond (full width half maximum), configured to deliver pulses with a length of not more than 1 picoseconds (full width half maximum), and/or configured to deliver pulses with a length selected for athermal ablation of ligamentum flavum. The endoscopic device may further include a standoff to enforce a minimum separation distance between the output of the optical fiber and a target surface, and/or a saline source coupled to a saline output at the first end of the body, where the saline output is directed toward the target at the output end of the optical fiber. The endoscopic device may further include a saline source coupled to a saline output at the first end of the body, the saline output directed toward the camera on the first end. The endoscopic device may further include a steering mechanism to control a direction of the output end relative to the body of the endoscopic device. The steering mechanism may include at least one of a magnet coupled to the body near the first end, a control wire coupled between the first end and the second end to control a curvature of the body, and one or more balloons coupled to an exterior of the body and inflatable to move the body of the endoscopic device within a surgical cavity. The steering mechanism may include a plurality of concentrically nested and curved tubes within the body operable to control a curvature of the body. The endoscopic device may further include a protective shield at the first end of the body positioned to protect an area around the target from optical energy delivered by the laser, a steering mechanism operable from the second end of the body to control a position of the protective shield around the target, and/or one or more extendable flaps on the first end of the body operable to displace tissue around the first end from the field of view of the camera.

### BRIEF DESCRIPTION OF THE DRAWINGS

The foregoing and other objects, features and advantages of the devices, systems, and methods described herein will be apparent from the following description of particular embodiments thereof, as illustrated in the accompanying drawings. The drawings are not necessarily to scale, emphasis instead being placed upon illustrating the principles of the devices, systems, and methods described herein.
Fig. 1 shows a device traversing a surgical canal.
Fig. 2 shows a device traversing a surgical canal.
Fig. 3 shows a device traversing a surgical canal including a control wire.
Fig. 4 shows a device traversing a surgical canal including magnetic control.
Fig. 5 shows a device traversing a surgical canal including a balloon.
Fig. 6 is a flowchart of a method for traversing a surgical canal.
Fig. 7 shows a surgical tool in an undeployed state.
Fig. 8 shows a surgical tool in a deployed state.
Fig. 9 shows a surgical tool in a deployed state.
Fig. 10 shows a deployment mechanism in an undeployed state.
Fig. 11 shows a deployment mechanism in a deployed state.
Fig. 12 shows a deployment mechanism in an undeployed state.
Fig. 13 shows a surgical tool with a flexible coating.
Fig. 14 shows a surgical apparatus.
Fig. 15 shows a surgical apparatus with a surgical tool on a distal end thereof.
Fig. 16 shows a surgical apparatus with a protective shield on a distal end thereof.
Fig. 17 shows an endoscopic body with a plurality of flaps in an undeployed state.
Fig. 18 shows an endoscopic body with a plurality of flaps in a deployed state.
Fig. 19 shows an endoscopic device for removal of ligamentum flavum.
Fig. 20 shows a fluence spatial pattern for a laser on a target surface.

### DETAILED DESCRIPTION

The embodiments will now be described more fully hereinafter with reference to the accompanying figures, in which preferred embodiments are shown. References to items in the singular should be understood to include items in the plural, and vice versa, unless explicitly stated otherwise or clear from the context. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth.

Recitation of ranges of values herein are not intended to be limiting, referring instead individually to any and all values falling within the range unless otherwise indicated herein, and each separate value within such a range is incorporated into the specification as if it were individually recited herein. The words "about," "approximately," or the like, when accompanying a numerical value, are to be construed as indicating a deviation as would be appreciated by one of ordinary skill in the art to operate satisfactorily for an intended purpose. Ranges of values and/or numeric values are provided herein as examples only, and do not constitute a limitation on the scope of the described embodiments. The use of any and all examples, or exemplary language ("e.g.," "such as," or the like) provided herein, is intended merely to better illuminate the embodiments and does not pose a limitation on the scope of the embodiments or the claims. No language in the specification should be construed as indicating any unclaimed element as essential to the practice of the embodiments.

In the following description, it is understood that terms such as "first," "second," "top," "bottom," "up," "down," "backward," "forward," and the like, are words of convenience and are not to be construed as limiting terms unless specifically stated to the contrary.

Described herein are devices, systems, and methods related to various surgical instruments and their applications in minimally invasive surgeries such as laser removal of ligamentum flavum from the spinal cavity as a surgical treatment for spinal stenosis. However, a person of ordinary skill in the art will recognize that other applications are possible and that applications of the systems and methods described herein are not limited to those explicitly described in this disclosure. For example, the devices, systems, and methods described herein may be used in other surgeries, particularly for surgeries involving removal of tissue with similar properties to ligamentum flavum. At the same time, the techniques disclosed herein may be adapted for removal of other tissue types through appropriate modifications of laser types, fiber types, drive techniques, and so forth. The other non-laser-specific tools and features may also have broad applicability in other minimally invasive procedures. Other uses outside of the field of surgery are also possible, including without limitation, other systems such as those used in the field of plumbing, electrical systems, military or law enforcement applications, and so forth (e.g., for the inspection or manipulation of industrial machines).

The systems, devices, and methods, described herein may be tailored for use with an endoscope. The endoscope may include one or more concentrically nested and curved tubes within the body of the endoscope such as those described in U.S. App. No. 14/550,436 filed on November 21, 2014. In another aspect, the body may have one or more pre-curved portions and/or adjustably bendable portions for adapting the shape of an endoscopic body-such as the body of an endoscope or the body of a cannula used to deliver the endoscope to a surgical site-to a surgical environment, e.g., a spinal canal. For example, an embodiment includes a flexible endoscopic body having a first end and a second end, and a tube within the flexible endoscopic body having an end proximal to the first end of the flexible endoscopic body with a predetermined radius of curvature and a predetermined stiffness, where the tube includes a hollow core and an exit from the hollow core for a surgical tool. This embodiment may also include a sleeve slidably disposed outside of and concentric with the tube, where the sleeve has a terminal portion proximal to the end of the tube with a stiffness greater than the predetermined stiffness of the tube and a radius of curvature greater than the predetermined radius of curvature of the tube. This embodiment may also a control mechanism proximal to the second end of the flexible endoscopic body with a control configured to change a curvature of the end of the tube by axially displacing the sleeve relative to the tube.

A variety of flexible tools for use in minimally invasive surgery and the like will now be described. The flexible tools may include an endoscope, a cannula, or any other endoscopic body suitable for use in a minimally invasive surgical procedure. It will be understood that at least two types of steering are contemplated herein. In one aspect, the shape of the endoscopic body itself is shaped to achieve a desired approach to a surgical site. This may include a compound curvature with a first curve having an apex or other similar surface designed to rest a portion of the endoscopic body on a safe location within a canal to provide a fulcrum (of sorts) for more stable and forceful manipulation of the distal end. A second curve in the compound curvature may incline a surgical tool away from the interior walls of the spinal canal and/or toward a target surgical site. A suitably configured compound curve can facilitate desired control of the distal end in a manner that permits an application of mechanical force at the distal end as desired or required during a procedure. A second type of steering relates more specifically to fine control or positioning of the distal end during a procedure. Thus, for example with the apex or other supporting surface positioned securely against an interior wall of the spinal canal, the distal end may be steered using a combination of a camera for visual navigation and one or more steering controls as contemplated herein. One useful example of a user control for steering is provided in the '436 patent, although other control interfaces and mechanisms may also or instead be used.

In general, a cannula is a tube that can be inserted into a surgical site, often for the delivery or removal of fluid, or for obtaining data. A cannula may also or instead surround the inner or outer surfaces of a needle or other surgical tool such as an endoscope. In the context of minimally invasive surgery or the like, a flexible cannula may be used for positioning and manipulation of a surgical tool around delicate or sensitive areas where contact should be avoided or minimized. To this end, a flexible cannula may bias instruments (e.g., long, flexible instruments or tools) into a preferred orientation within a surgical site or the like. In this context, various devices, systems, and methods are described for deploying a flexible tool (e.g., a cannula) within a cramped or convoluted channel (e.g., a surgical site) so as to avoid or minimize contact with sensitive areas (e.g., soft tissue) and/or to provide a safe and secure resting place near a surgical site to stabilize the distal end and provide a platform for further downstream mechanical steering and other manipulations. Thus, some of the techniques contemplated herein, particularly those relating to curvature of an endoscopic device may be equally applicable to an endoscope, a cannula, or some combination of these or any other endoscopic or laparoscopic body.

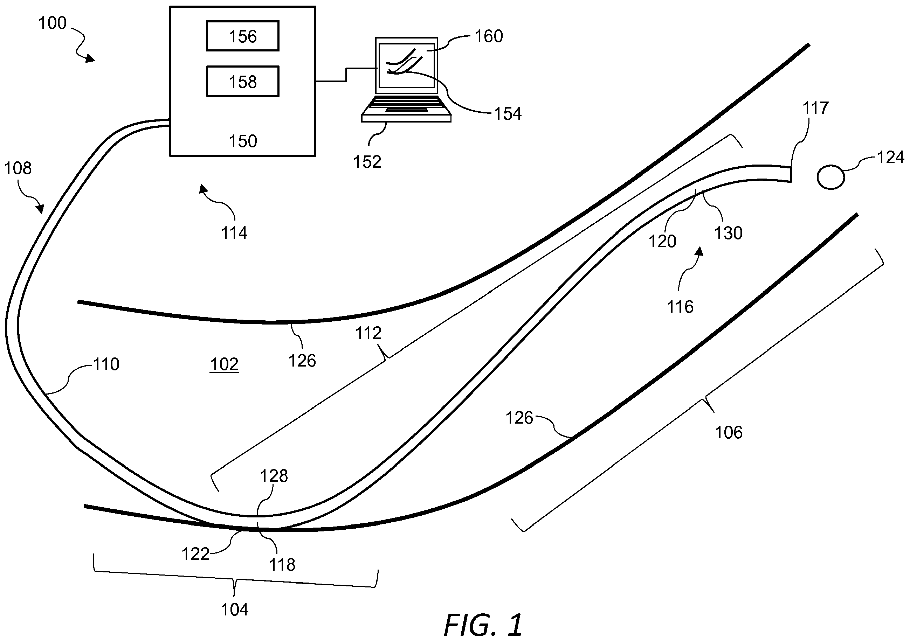

Fig. 1 shows a device traversing a surgical canal. The device 100 may be configured for deployment in a relatively convoluted or cramped channel (e.g., a surgical canal 102 as shown in the figure), such that contact with sensitive or delicate areas of the channel are minimized or avoided.

The surgical canal 102 may for instance be a spinal canal of a patient, where a portion of the device 100 is configured for insertion past the sacral hiatus and into the spinal canal. The surgical canal 102 may include a supporting tissue region 104 and a soft tissue region 106. The supporting tissue region 104 may be a relatively hard tissue region as compared to the soft tissue region 106. The supporting tissue region 104 may include, for example, one or more of epidural fat, a disc, a ligamentum flavum, bone, and so forth. The soft tissue region 106 may be considered to be any relatively delicate or sensitive region of a surgical site, i.e., where contact is advantageously minimized or avoided. More generally, the surgical canal 102 may be characterized by numerous types and positions of tissues, including any number of supporting tissue regions 104 and soft tissue regions 106, although one region of each tissue type is specifically identified here for purposes of describing corresponding positions of a surgical tool.

The surgical canal 102 may be a portion of a human spinal canal such as the sacral canal of a human spinal column or any other similarly constrained anatomical space. For the spinal canal the surgical entry point may correspond to the sacral hiatus, the supporting tissue region 104 may correspond to a region of bone tissue (e.g., along the dorsal portion of the spinal column), and the soft tissue region 106 may include exposed nerve roots, e.g. of the sacral nerves. As discussed herein, other surgical contexts or non-surgical contexts are possible.

The device 100 may include a surgical tool 108 having a body 110 with a compound curve 112 included therein. The surgical tool 108 may further include a control end 114 positioned outside the patient when the surgical tool 108 is positioned for use, and a distal end 116 configured to be located inside of the surgical canal 102 (e.g., spinal canal) when the surgical tool 108 is positioned for use. In general, the device 100 may be configured such that it avoids contact with the soft tissue region 106 of the surgical canal 102. Some tools of the prior art appear to facilitate some curvature, but they are generally pre-curved to follow the surgical canal, or physically deformed by the canal during entry. These deformations are generally made without regard to the location of resulting contact points or the approach trajectory of any surgical tools toward the ultimate surgical target. This can lead to numerous negative side effects such as contact with soft tissue or other sensitive regions, as well as constrained access to the target site by the distal end 114.

By imposing a suitable compound curvature, these difficulties can be alleviated. For example, a bend of the curve may be positioned to support mechanical leverage on the distal end 114, either to apply pressure to a target with the end or to maintain a desired separation between the distal end 114 and a target surface such as an interior wall of the surgical canal 102. In one aspect, the compound curve 112 may be configured to remain in contact with the dorsal wall of the spinal canal along a substantial portion of its length, thus maintaining the distal end 114 in a position away from the opposing wall.

The surgical tool 108 may include without limitation an endoscope, a cannula for an endoscope, a trocar, and so forth. The surgical tool 108 may also or instead include any relatively long, flexible instrument capable of being guided through a convoluted or cramped channel. The surgical tool 108 may thus include any tool classified in a relatively broad category of tools with steerable tips that can be controllably positioned within a body cavity or the like after positioning through an endoscope.

The surgical tool 108 may also or instead include a laser, an optical fiber, a camera, a cutting tool, an ablation tool, a grasping tool, a hooking tool, a piercing tool, a locking tool, a shield, a guiding mechanism, a material or fluid delivery system (e.g., medicinal or otherwise), a sampling device or transducer, a sensor, and so forth. For example, in an aspect, the device 100 includes a surgical laser and an optical fiber positioned to transmit optical energy from the surgical laser through the body 110 and to a distal tip 117 on the distal end 116. In another aspect, the device 100 includes an electrosurgical radio frequency device coupled to the distal end 116 and operable for radio frequency ablation of a surgical target 124. These components (e.g., the laser, the cutting tool, the electrosurgical radio frequency device, etc.) may be engaged on the distal end 116 of the surgical tool 108.

The body 110 may be shaped and sized for insertion past a sacral hiatus and into a spinal canal of a patient. In an aspect, the body 110 has an outside diameter not greater than three millimeters to facilitate access through this passage. The body 110 may include one or more pathways or working channels disposed therein, where tools or other objects or couplings (e.g., electrical wires for data and power, mechanical wires for steering and other manipulations, and so forth) can be deployed. The body 110 may be flexible and steerable. The body 110 may include a predetermined stiffness that may be adapted through an adjustment of components included therein (e.g., concentric tubes).

The compound curve 112 may be disposed in the body 110 of the surgical tool 108. The compound curve 112 may include at least a first bend 118 and a second bend 120.

The first bend 118 may be positioned to provide a point of contact 122 between the surgical tool 104 and the supporting tissue region 104 of the surgical canal 102 when the distal end 116 (i.e., a distal tip 117 of the distal end 116) is positioned for a procedure on a surgical target 124 within the surgical canal 102 (e.g., spinal canal). In this manner, the first bend 118 of the compound curve 112 may provide a mechanical support (or anchor) for a position of the distal tip 117. The second bend 118 of the compound curve 112 may be positioned to direct the distal end 116 (and/or the distal tip 117) toward the surgical target 124 when the surgical tool 108 is placed for use.

In an aspect, the first bend 118 has a predetermined stiffness. The predetermined stiffness may be selected to deform to a native curvature of the surgical canal 102 (e.g., spinal canal) in order to retain adequate mating contact between the body 110 and a wall 126 of the surgical canal 102 to securely support other manipulations within the surgical canal 102. The deformation to the native curvature of the surgical canal 102 may occur in a region between a first apex 128 of the first bend 118 and a second apex 130 of the second bend 120 in order to retain the distal end 116 (or distal tip 117 thereof) in a fixed position relative to the surgical target 124. The predetermined stiffness may also or instead be selected to retain the second apex 130 in contact with the wall 126 of the surgical canal 102. It will be understood that an "apex" as used mathematically may connote one or more exact locations in a particular shape or coordinate system. The usage here is intended more generally to refer to any region along a length of the surgical tool 110 that is shaped to provide mechanical contact, support, or steering as contemplated herein within the context of the surgical canal 102.

The control end 114 may include a controller 150 coupled in a communicating relationship with the device 100 and a computing device 152. The controller may include a processor 156 and a memory 158 configured to perform the control operations described herein.

In general, the controller 150 may be operable to control the device 100. The controller 150 may include any combination of software and/or processing circuitry suitable for controlling the various components of the system 100 described herein including without limitation processors, microprocessors, microcontrollers, application-specific integrated circuits, programmable gate arrays, databases, and any other digital and/or analog components, as well as combinations of the foregoing, along with inputs and outputs for transceiving control signals, drive signals, power signals, sensor signals, and the like. In one aspect, the controller 150 may include a microprocessor or other processing circuitry (e.g., the processor 156) with sufficient computational power to provide related functions such as executing an operating system, to provide a graphical user interface (e.g., to a display coupled to the controller 150 or another component, e.g., the computing device 152), to set and execute rules and instructions for operation of the device 100, and so forth.

The computing device 152 may include any device or combination of devices operated by users to manage, monitor, communicate with, control, or otherwise interact with the device 100 or controller 150. This may include desktop computers, laptop computers, network computers, tablets, smart phones, smart watches, personal digital assistants, or any other device that can participate with the elements contemplated herein. The computing device 152 may include a user interface 160 such as a graphical user interface for displaying a model 154 of the surgical canal 102. In an aspect, the model 154 includes one or more of a two-dimensional image of the surgical canal 102, a cross-section of the surgical canal 102, a three-dimensional image of the surgical canal 102, and so on. The model 154 may be a model specific to a surgical patient, such as from a magnetic resonance image, x-ray image, computerized axial tomography scan, x-ray computed tomography image, or any other two-dimensional or three-dimensional image of the relevant portions of the patient's anatomy. In another aspect, a generalized model of the relevant geometry may be employed. In the latter case, it may be more difficult to properly position the compound curvature of the body, and visual guidance during insertion may be used to supplement the initial compound curve with modifications as appropriate during a procedure, either via retraction and manual re-shaping, or through curvature control mechanisms such as those contemplated herein.

The user interface 160 may also or instead include a text or command line interface, a voice-controlled interface, and/or a gesture-based interface. In general, the user interface may create a suitable display on the computing device 152 for user interaction. The user interface may be maintained by a locally executing application on the computing device 152 that receives data from the device 100 or other resources. In other embodiments, the user interface may be remotely served and presented on the computing device 152, such as where a web server provides information through one or more web pages or the like that can be displayed within a web browser or similar client executing on the computing device 152.

Other components, e.g., other hardware, may also be included such as input devices including a keyboard, touchpad, mouse, switches, dials, buttons, sensors, and the like, as well as output devices such as a display, a speaker or other audio transducer, light emitting diodes, and the like. Other components may also or instead include a variety of cable connections and/or hardware adapters for connecting to, e.g., external computers, external hardware, external instrumentation or data acquisition systems, and the like.

The controller 150 may also or instead include a manual controller, e.g., where the controller 150 is integrated into or includes a handheld device, with physical movement of a joystick or buttons on the controller 150 creating corresponding movements of the surgical tool 108.

In use, the device 100 may be deformed prior to deployment such that when the device 100 is in a deployed state (i.e., when the distal tip 117 of the surgical tool 108 is within operating range of the surgical target 124), the point of contact 122 lies on the supporting tissue region 104. This deformation may be carried out based on one or more models 154 (or images, e.g., x-ray images, MRI images, etc.) of the surgical canal 102. In some implementations, rather than deforming an existing device 100, a device may be fabricated in the first instance with the geometry described herein, based on a model 154 of the surgical canal 102. In any case, this configuration during deployment may mitigate or eliminate negative side effects of compressing, scratching, or otherwise contacting any exposed soft tissue region 106 with the surgical tool 108.

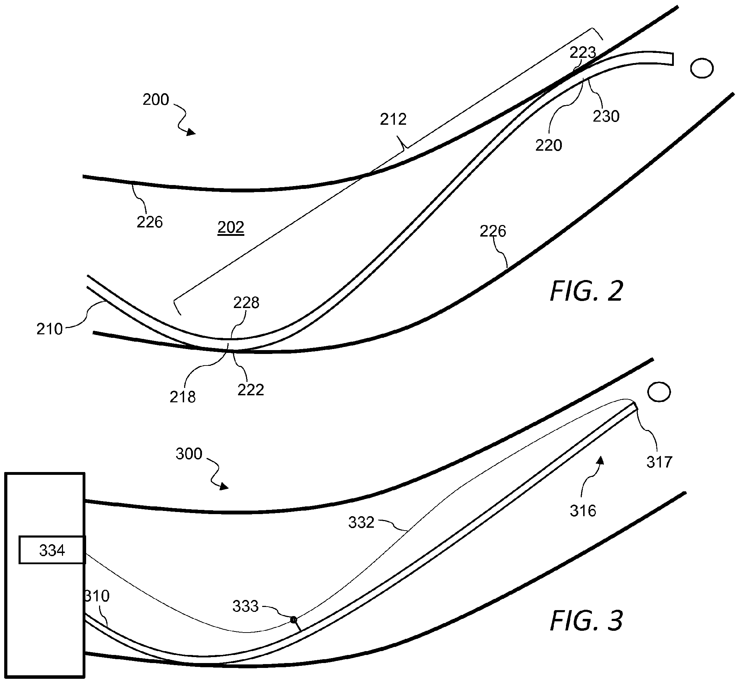

Fig. 2 shows a device traversing a surgical canal. The device 200 in this figure is similar to the device shown above, but it includes a body 210 with a compound curve 212 where the first apex 228 of the first bend 218 and the second apex 230 of the second bend 220 both contact the wall 226 of the surgical canal 202. Specifically, the first apex 228 contacts the wall 226 of the surgical canal 202 at a first point of contact 222 and the second apex 230 contacts the wall 226 of the surgical canal 202 at a second point of contact 223. This may be caused by the predetermined stiffness of the body 210 (e.g., the compound curve 212) retaining both apexes 228, 230 in contact with the wall 226 of the surgical canal 202.

While the figures above show one point of contact and two points of contact with the wall of the surgical canal, respectively, one skilled in the art will recognize that more points of contact are possible. For example, an implementation can include more than two bends in the compound curve, or the compound curve may be adjusted to follow portions of an interior of the surgical canal 202, e.g., to contact or remain in close proximity to a region of the surgical canal 202 along a length of the body 210. There may also or instead be more than two points of contact with the wall of the surgical canal, e.g., one or more points of contact associated with each bend included in the compound curve. As such, any number of possible bends and corresponding points of contact are possible. In an aspect, all points of contact are configured to be located in the supporting tissue region(s). However, although it may generally be desirous to avoid contact with the soft tissue region as described herein, the one or more points of contact associated with the one or more bends included in the compound curve may instead be disposed in the soft tissue region, or any combination of the supporting tissue region and the soft tissue region, or elsewhere in the surgical canal 202.

In an aspect, depending on the geometry of the surgical canal, it may not be possible to arrange for each point of contact to lie on the supporting tissue region. In this case, refinements of the above-described techniques may be employed, where two such examples are provided below.

As a first example, the points of contact may be prioritized in some fashion, and the main point or points of contact (i.e., those with the highest priority) may be placed over the supporting tissue region. In some implementations, the points of contact may be prioritized based on the expected pressure at the point of contact when the device is in a deployed state (i.e., highpressure points are given high priority). The expected pressure at a point of contact may be determined by a model (e.g., a mathematical model, a physical prototype, a combination thereof, etc.) or any other means. In some implementations, the points of contact may be prioritized according to anatomical significance with respect to a particular patient. Other ways to prioritize points of contact are also or instead possible.

As a second example, for some types of soft tissue there may be an acceptable pressure threshold below which negative side effects may be minimized or eliminated. In general, the acceptable pressure threshold may depend on the type of soft tissue contacted by the device. When one or more points of contact must be disposed on soft tissue, the techniques described herein can be used to distribute the pressure from the device over the soft tissue region such that the overall pressure is below an acceptable threshold, to the extent possible. Thus a curve of the device may be designed to rest at a particular region within the wall, may have a shape selected to distribute a contact surface along a greater portion of its length, a stiffness adapted to conform to the surgical canal and thus distribute a contact point along a greater portion of its length, and so forth.

Fig. 3 shows a device traversing a surgical canal including a control wire. Specifically, the device 300 in this figure is similar to the devices described above, but the device 300 includes a control wire 332 coupled between the control end and the distal end 316 to control a positon of the distal end 316 (e.g., distal tip 317) relative to the body 310. The control wire 332 may be mechanically coupled to the body 310 of the device 300 (e.g., at one or more anchor points 333) to control movement of the device 300, e.g., to deflect the device 300 away from soft tissue, or otherwise mitigate the negative scenarios described above.

The one or more anchor points 333 may be located anywhere along the body 310 of the device 300, e.g., at the distal end 316 thereof. The control wire 332 may also or instead be mechanically coupled to the body 310 of the device 300 via various other couplings along the length of the body 310. Such couplings can include, e.g., loops through which the control wire 332 may pass, or the like.

The control wire 332 may also or instead be mechanically coupled to a control unit 334 or controller at the proximal end of the device 300 (e.g., similar to the controller described above). In some implementations, the control unit 334 may include a dial, trigger, or similar control that allows an operator to apply various pre-selected degrees of tension to the control wire 332. In some implementations, the control unit 334 may allow the operator to apply a continuously variable degree of tension in the control wire 332. Applying tension to the control wire 332 may advantageously allow an operator of the device 300 to alter its shape. In the figure, for example, the device 300 is illustrated in a state with little or no tension in the control wire 332. By increasing the tension in the control wire 332, the device 300 may be deflected away from soft tissue, thereby avoiding any negative consequences associated with contacting soft tissue.

More generally, a variety of techniques are known for controlling shape of an elongated member using one or more control wires (according to the degrees of freedom desired for positioning), any of which may be adapted for use with a system as contemplated herein. Also, it will be appreciated that while the control wire 332 is illustrated outside of the body 310 in order to illustrate function, the control wire 332 may be usefully positioned inside the body 310 in order to avoid exposure to the anatomy and tissue within the spinal canal.

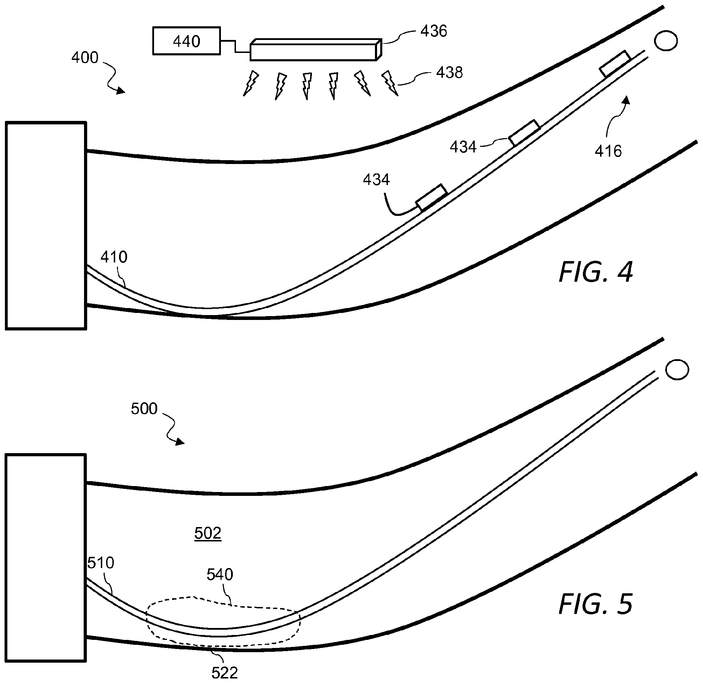

Fig. 4 shows a device traversing a surgical canal including magnetic control. Specifically, the device 400 in this figure is similar to the devices shown above, but the device 400 includes one or more a magnets 434 coupled to the body 410. The magnets 434 may be disposed at or near the distal end 416 of the body 410, or elsewhere along the body 410. The magnets 434 may work in conjunction with an external magnet 436 that is configured to apply a magnetic field 438 to control the device 400. For example, in an implementation, prior to or during deployment, the magnetic field 438 may be provided by the external magnet 436 to cause the magnets 434 to experience a force in a desired direction, e.g., in a direction to deflect the device 400 away from soft tissue. In some implementations, the magnitude and/or direction of the magnetic field 438 may be controllable via an external controller 440 (e.g., similar to the controllers described above).

Fig. 5 shows a device traversing a surgical canal including a balloon. Specifically, the device 500 in this figure is similar to the devices shown above, but the device 500 includes one or more balloons 540 or other elastic or inelastic inflatable membranes coupled to an exterior of the body 510 and inflatable to move the body 510 within the surgical canal 502. The balloon 540 may act as an element to control the device 500 and/or prevent contact with soft tissue or the like. The balloon 540 may be inflated to a desired degree before or during deployment of the device 500. When inflated, the balloon 540 may be operable to distribute a contact force of the body 510 of the device 500 at the point of contact 522, resulting in an overall lower pressure at the point of contact 522. In some implementations, the balloon 540 may be placed at a location along the body 510 of the device 500 where a point of contact 522 is expected to occur when the device 500 is in a deployed state. This location may be identified by a mathematical model (e.g., as a function of a patient's height, weight, etc.) and/or from inspection of one or more images of the surgical canal 502. Thus, the balloon 540 may be used to establish a contact point (e.g., the point of contact 522) for applying leverage along a length of the body 510 of the device 500 in order to move, position, or stabilize an extended end of the surgical tool near the surgical target or to otherwise manipulate or position the device 500 within the surgical canal 502. In general, the balloon 540 may be inflated, deflated, and moved along the length of the body 510 of the device 500 as appropriate for a particular use. In some implementations, the balloon 540 may be controllable via an external controller (e.g., similar to the controllers described above).

As referenced above, the devices with compound curves may further include one or more concentrically nested and curved tubes within the body. In an aspect, the concentrically nested and curved tubes are operable to control a curvature of at least one of the first bend and the second bend.

In embodiments, various combinations of steering, positioning, and control techniques may be employed. Thus a device may use any combination of the techniques describe above-magnets, control wires, balloons, and concentric tubes-as well as any other suitable techniques and any combination of the foregoing. Similarly, one technique may be used to achieve a controllable, aggregate compound curve in the body 510 of the device 500 while a second technique may be used for fine control of a positon or orientation of the distal end where a surgical tool or the like is mounted. All such combinations and variations are intended to fall within the scope of this disclosure.

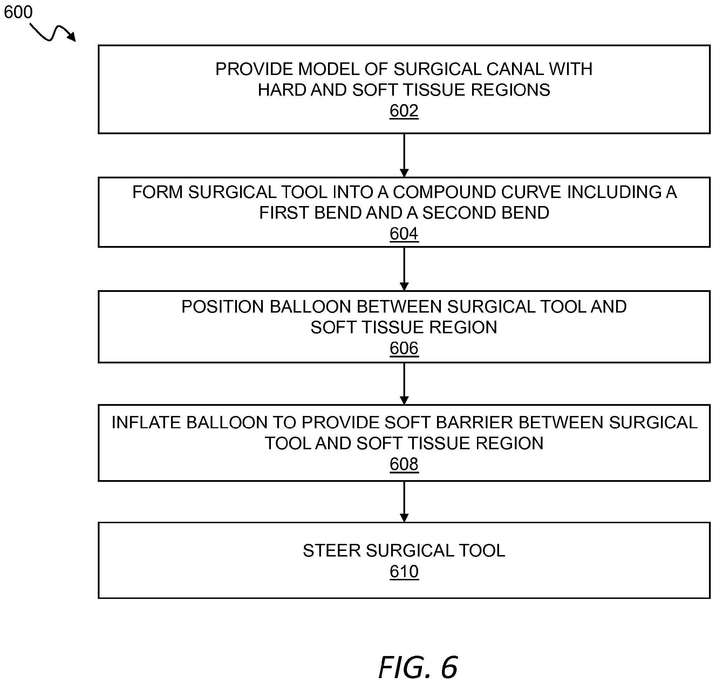

Fig. 6 is a flowchart of a method for traversing a surgical canal.

As shown in step 602, the method 600 may include providing a model of a surgical canal of a patient. The surgical canal may include a surgical entry point, a supporting tissue region, a soft tissue region, and a surgical target. The surgical canal may also include a curvature. The model may include any of the models described herein including without limitation a two-dimensional or three-dimensional image of the surgical canal of a particular patient or a generalized model suitable for approximating an expected shape.

As shown in step 604, the method 600 may include forming a surgical tool having a distal tip into a compound curve. The surgical tool may be any as described herein, e.g., at least one of an endoscope and a cannula for the endoscope.

The compound curve may include a first bend positioned to provide a point of contact between the surgical tool and the supporting tissue region of the surgical canal when the distal tip is positioned for a procedure on the surgical target. The first bend may provide a mechanical support for a position of the distal tip. The compound curve may further include a second bend positioned to direct the distal tip toward the surgical target. In an implementation, the second bend may also or instead provide a supplemental point of contact between the device and a wall of the surgical canal to further support and stabilize the device for a surgical procedure.

As shown in step 606, the method 600 may include positioning a balloon at a location between the surgical tool and the soft tissue region. This may include moving the body of the surgical device until a fixed, attached balloon is in a suitable position, or moving a balloon along the body until it is positioned in an appropriate location.

As shown in step 608, the method 600 may include inflating the balloon to provide a soft barrier between the surgical tool and the soft tissue region. Regardless of how positioned, the balloon may more generally be inflated, deflated, or otherwise adjusted to achieve desired mechanical results for the surgical tool or body that is being positioned within the spinal canal.

As shown in step 610, the method 600 may include steering the surgical tool. This may include fine steering as described above, e.g., controlling a position of the distal tip of the surgical tool to achieve a desired position relative to a surgical target. This may also include general steering via control of the bend of the surgical tool or an end thereof to positon the overall device at a desired location within the spinal canal or other surgical cavity. Thus, steering the distal tip of the surgical tool may include steering the distal tip toward the surgical target, steering the distal tip away from the soft tissue region, or otherwise steering the distal tip or a distal end of the surgical tool, or the body of the surgical tool to effectuate an intended surgical operation. Steering may be performed using any of the techniques described herein. Thus for example, steering the distal tip may include steering the distal end with a wire mechanically coupled to the surgical tool from a control end to the distal tip. Steering the distal tip may also or instead include applying an external magnetic field to impart a force to move one or more magnets coupled to the surgical tool. Steering the distal tip may also or instead include rotating or translating one or more concentrically nested tubes within the surgical tool having different curvatures or stiffnesses.

Another implementation of a tool (e.g., a flexible tool) for use in minimally invasive surgery and the like will now be described, where the tool includes a surgical tool with flaps, e.g., for mitigating complications presented by obstructions encountered during a surgical procedure.

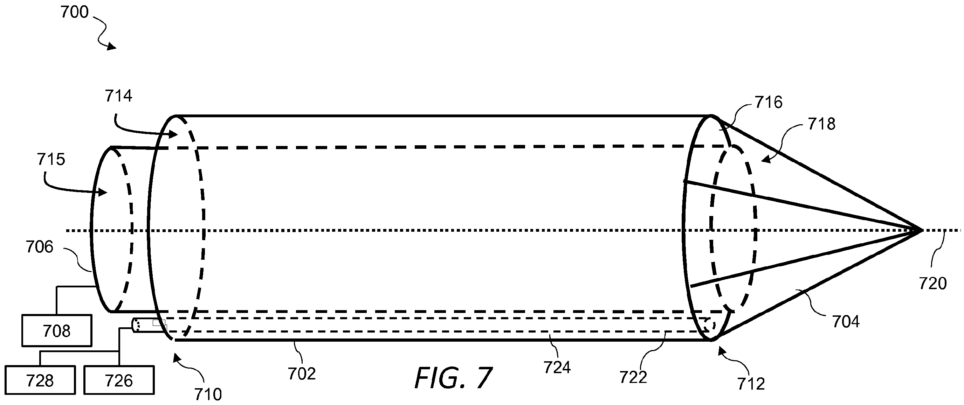

Fig. 7 shows a surgical tool in an undeployed state. The surgical tool 700 may include a device configured for deployment in a surgical site containing loose matter. The surgical tool 700 may be configured to mitigate problems caused by obstructions near a surgical target.

The surgical tool 700 may include a first tube 702, a plurality of flaps 704, a second tube 706, and a controller 708. The plurality of flaps 704 may also be referred to herein as "extendable flaps" to describe the ability to extend outward and away from the body of the surgical tool 700 as described herein.

The first tube 702 may include a proximal end 710, a distal end (i.e., the first distal end 712 shown in the figure), a hollow core 714, and an exit 716 from the hollow core 714 at the first distal end 712. The first tube 702 may thus include a hollow substantially cylindrical structure with the hollow core 714 formed therein, where the second tube 706 is disposed within the hollow core 714 of the first tube 702. In an aspect, the second tube 706 is also hollow, and thus includes its own inner hollow core 715.

In addition to the hollow core 714 of the first tube 702 and the inner hollow core 715 of the second tube 706, the surgical tool 700 may include a second hollow core 724 formed by the volume disposed between the interior of the first tube 702 and the exterior of the second tube 706, i.e., the void between the tubes 702, 706, or at any other suitable location and having any other suitable size for an intended use. The second hollow core 724 may be parallel to one or more of the hollow core 714 of the first tube 702 and the inner hollow core 715 of the second tube 706. The second hollow core 724 may instead be formed by one or more of the first tube 702 and the second tube 706 otherwise including pathways forming a plurality of additional hollow cores therein, e.g., for a plurality of supplemental tools.

In the surgical tool 700, the proximal end 710 may include or be configured for access to controls for manipulation of various tools during a surgical procedure. This may, for example, include controls for movement or function of components on the distal end 712. For example, the proximal end 710 may include the controller 708 and/or one or more mechanical or electromechanical elements for manipulating a body of the surgical tool 700 during a surgical procedure.

The first distal end 712 may be configured for insertion into a surgical site. As such, when in an undeployed state, the first distal end 712 may be sized and shaped for smoothly traversing a surgical canal.

The plurality of flaps 704 may be coupled to the first distal end 712 of the first tube 702. In an aspect, the plurality of flaps 704 are integrally formed with the first tube 702. Alternatively, in some implementations, the plurality of flaps 704 are separately formed from, but mechanically coupled to, the first tube 702. In an aspect, each of the plurality of flaps 704 bears a smoothly contoured shape free of sharp edges and corners. Such a construction may mitigate the risk of puncturing or scratching sensitive tissue during the deployment of the surgical tool 700 to a surgical site and the subsequent deployment (e.g., by opening or spreading) of the plurality of flaps 704 included thereon.

The second tube 706 may be slidably disposed within and concentric to the first tube 702. In an aspect, both the first tube 702 and the second tube 706 are slidably disposed relative to one another, e.g., along a longitudinal axis 720 of the tubes 702, 706. The second tube 706 may include a distal end, i.e., the second distal end 718 shown in the figure. The second distal end 718 may include an exit (i.e., in an embodiment where the second tube 706 is hollow) or a functional tool disposed thereon. Thus, in an embodiment where the second tube 706 is hollow, the inner hollow core 715 may include a pathway through which surgical instruments (e.g., cameras, lasers, therapeutic agents, or the like) may pass. In this manner, the second tube 706 may itself be a primary tool for use in the overall surgical tool 700 shown in the figure, or it may contain a primary tool for use in the overall surgical tool 700.

It will be appreciated that the first tube 702 and the second tube 706 may either or both contain a predetermined curvature and stiffness as contemplated, for example, in U.S. Pat. App. No. 14/550,436. The operation of this nested, concentric tube system for steering an endoscopic body is not described herein in detail, but is described by way of numerous examples in the '436 application. For purposes of adequate description, it is simply noted here that the first tube 702 and the second tube 706 may structurally be two concentric nested tubes with predetermined stiffness and curvature operable to control a curvature of an endoscopic body (such as any of the endoscopic bodies contemplated herein) containing the tubes 702, 706.

The controller 708 may be operable to activate the controllable aspects of the surgical tool 700 described herein, such as the relative motion of the tubes, the articulation of the flaps, and so forth. At an appropriate moment (e.g., when the surgical tool 700 and instruments contained therein are near the surgical target), the controller 708 may be used to transition the surgical tool 700 from the undeployed state to the deployed state. Thus, in an aspect, the controller 708 is operably configured to move the plurality of flaps 704 relative to the first tube 702, such as by axially sliding the first tube 702 and the second relative to one another. For example, the controller 708 may move the plurality of flaps 704 between an undeployed state where the plurality of flaps 704 are contained within a cross section normal to the longitudinal axis 720 of the first tube 702 and a deployed state where the plurality of flaps 704 are at least in part disposed outside of the cross section to provide a mechanical separation between the distal end 712 of the first tube 702 (or other portion of the surgical tool 700) and tissue surrounding a surgical site.

The controller 708 may be disposed at a proximal end 710 of the device, e.g., external to the surgical site. The controller 708 may be the same or similar to the any of the controllers described herein.

In the undeployed state shown in the figure, the plurality of flaps 704 may be generally gathered around the longitudinal axis 720. In some implementations, the plurality of flaps 704 in the undeployed state may collectively block access to the interior of the tubes, i.e., the hollow core 714, the inner hollow core 715, and/or the second hollow core 724. This may be useful, among other reasons, to protect the contents of the hollow cores while the surgical tool 700 is being guided to a surgical target. Alternatively, in some implementations the plurality of flaps 704 in the undeployed state need not block access to the interior of the tubes. This may be useful, among other scenarios, when the interior of the tubes includes a camera used to aid navigation of the surgical tool 700 to the surgical target. In the deployed state (e.g., shown in the figure described below), the plurality of flaps 704 may controllably flare out from the longitudinal axis 720. This flaring action may be advantageously deployed, among other ways, to clear loose matter lying between the surgical tool 700 and the surgical target.

The surgical tool 700 may further include one or more supplemental tools 722. As shown in the figure, the supplemental tool 722 may be disposed in the second hollow core 724 of the first tube 702. A supplemental tool 722 may also or instead be disposed in the hollow core 714 or any other suitable location. Regardless of the tube or core in which it's disposed, the supplemental tool 722 may be configured such that it is generally disposed at the distal end 712 of the first tube 702 and, where the supplemental tool 722 is an active tool with control aspects, coupled to a second controller 726 at the proximal end 710 of the first tube 702. The second controller 726 may be the same or similar to the any of the controllers described herein, and in an aspect, the second controller 726 is part of the controller 708 described above (i.e., there is one controller performing a plurality of control functions).

The supplemental tool 722 may include without limitation a laser, an electrosurgical radio frequency device, an imaging element, a saline wash, an illumination source, a mechanical locking mechanism, a piercing tool, a cutting tool, a grasping tool, a hooking tool, a protective shield, concentrically nested and curved tubes, an endoscope, and so forth. In an aspect, the supplemental tool 722 includes a laser surgical tool disposed at the distal end 712 of the first tube 702 and coupled to the second controller 726 at the proximal end 710 of the first tube 702. In another aspect, the supplemental tool 722 includes an electrosurgical radio frequency device disposed at the distal end 712 of the first tube 702 and coupled to the second controller 726 at the proximal end 710 of the first tube 702.

As discussed above, the supplemental tool 722 may include an imaging device and a saline wash or similar. For example, in an aspect, the surgical tool 700 includes an imaging element disposed at the distal end 712, and the one or more supplemental tools 722 includes a saline wash sourced through the second hollow core 724 and directed at the imaging element. In an aspect, the imaging element is included within the second tube 706 or is integral with the second tube 706. The imaging element may include at least one of a digital camera and an optical fiber for optically transmitting an image at the distal end 712 of the surgical tool 700 to the proximal end 710.

The supplemental tool 722 may include an illumination source at the distal end 712 coupled to a power source 728 and a control source (e.g., the second controller 726) at the proximal end 710 of the first tube 702.

In another aspect, the supplemental tool 722 may include a surgical stapler, suturing device, or the like to close openings that are intentionally or unintentionally formed in tissue during a procedure. Similarly, a surgical glue dispenser or the like may be provided for intraoperative guided tissue repair. The supplemental tool 722 may also include a pincer or the like to hold tissue together during a gluing, stapling, or suturing process. A pincer, grasper, tissue hook, gem holder, or the like may also or instead be used to secure the dura mater or other tissue during a procedure, or a number of such devices may be deployed around a perimeter of the insertion end of an endoscopic tool to secure the tool in a desired location during a procedure.

One skilled in the art will recognize that the supplemental tool 722 discussed herein may also or instead be included in or connected to the first tube 702 or the second tube 706 described above. Thus the surgical tool 722 may also or instead include any of the passive tools mechanically coupled to the distal end 720 as described below.

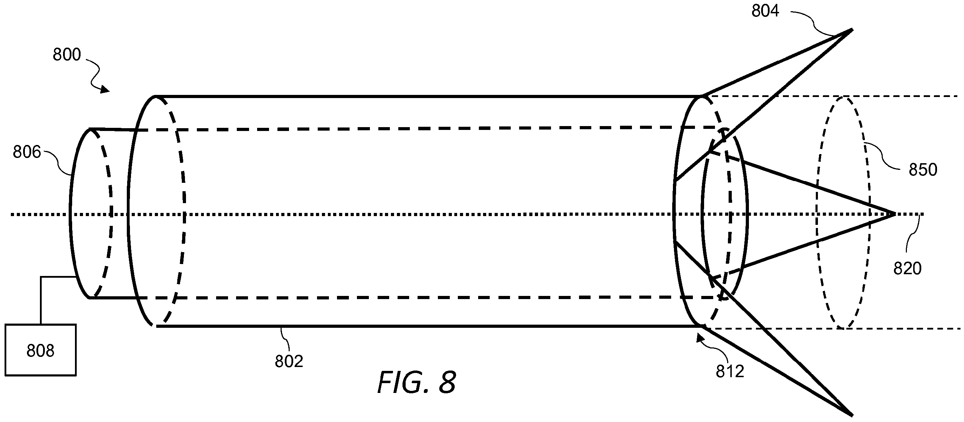

Fig. 8 shows a surgical tool in a deployed state. Similar to the tool described above, the surgical tool 800 may include a first tube 802, a plurality of flaps 804, a second tube 806, and a controller 808. Specifically, in the figure, the plurality of flaps 804 are in a deployed state where they are at least in part disposed outside of a cross section 850 normal to a longitudinal axis 820 of the first tube 802 to provide a mechanical separation between the distal end 812 of the first tube 802 (or other part of the surgical tool 800) and tissue surrounding a surgical site.

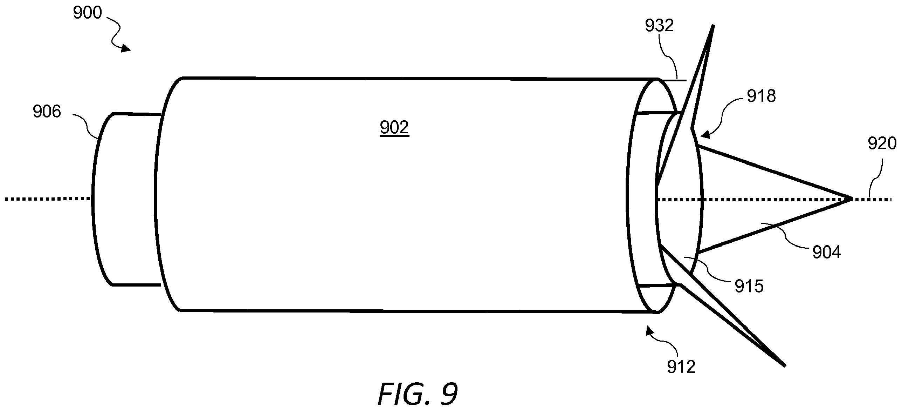

Fig. 9 shows a surgical tool in a deployed state. The surgical tool 900 shown in this figure is similar to those described above, but the surgical tool 900 has the plurality of flaps 904 disposed on the distal end 918 of the second tube 906 rather than the distal end 912 of the first tube 902. Specifically, the surgical tool 900 may include a controller operably configured to move the plurality of flaps 904 relative to the second tube 906 between an undeployed state where the plurality of flaps 904 are contained within a cross section normal to a longitudinal axis 920 of the second tube 906 and a deployed state where the plurality of flaps 904 are at least in part disposed outside of this cross section to provide a mechanical separation between the distal end 918 of the second tube 906 (or other part of the surgical tool 900) and tissue surrounding a surgical site. It will be understood that the features discussed above (with reference to the other figures) as being disposed on either the first tube or the second tube may thus be switched in the embodiment shown in this figure.

Additionally, as shown in the figure, in an aspect, the surgical tool 900 may include a protective shield 932 at the distal end 912 of the first tube 902. The protective shield 932 may be formed of a material selected to protect a surgical site from laser energy provided by a surgical laser operating at the distal end 912 of the first tube 902. In an embodiment, the protective shield 932 may also or instead be provided by the plurality of flaps 904 themselves, and thus no additional component is provided.

The plurality of flaps 904 shown in the figure may block access to the hollow core 915 in the undeployed state. Similarly, in the undeployed state, the plurality of flaps 904 may be contained at least in part within the first tube 902. Otherwise, the surgical tool 900 may include similarities to the structures described above.

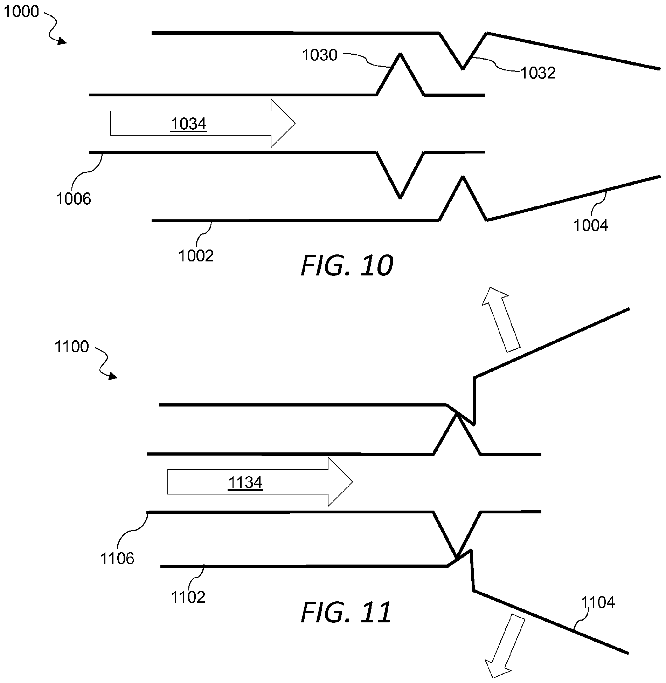

Fig. 10 shows a deployment mechanism in an undeployed state. The deployment mechanism 1000 may be used in conjunction with the surgical tools described above. For example, the deployment mechanism 1000 may be implemented on a surgical tool having a first tube 1002, a plurality of flaps 1004, and a second tube 1006.

As shown in the figure, the second tube 1006 may include a plurality of elements 1030 such as tabs, protrusions or the like that each mechanically mate with a corresponding element 1032 on one of the plurality of flaps 1004. The elements 1030, 1032 may be shaped such that sliding the second tube 1006 in a first direction (as indicated by the arrow 1034) relative to the first tube 1002 causes the plurality of elements 1030 to engage the plurality of flaps 1004 and move each of the plurality of flaps 1004 from the undeployed state to the deployed state (see figure described below).

Conversely, sliding the second tube 1006 in a second direction (e.g., opposite to the first direction) may disengage the plurality of elements 1030 from the plurality of flaps 1004. Further, an interior surface of the first tube 1002 may urge the plurality of flaps 1004 into the undeployed state, e.g., due to an elasticity or stiffness of the first tube 1002, or a spring, hinge, living hinge or the like formed into the first tube 1002 or coupled between the first tube 1002 and each of the flaps 1004. In another aspect, a similar hinging or rotational motion may be obtained from additional mechanical elements disposed on the interior surface of the first tube 1002 forcefully pulling the plurality of flaps 1004 into the undeployed state. In an aspect, each of the plurality of flaps 1004 is spring-biased toward the deployed state and the first tube 1002 retains the plurality of flaps 1004 in the undeployed state when the second tube 1006 is in its undeployed position (i.e., as shown in the figure).

The plurality of elements 1030 on the second tube 1006 and corresponding elements 1032 on the plurality of flaps 1004 are shown in the figure as corresponding projections, but one skilled in the art will recognize that these may also or instead include other cooperating elements (mechanical or otherwise). For example, these elements 1030, 1032 may include without limitation one or more of hooks, magnets, sliders, notches, ridges, grooves, and so forth. In some implementations, the end of one of the tubes or the flaps themselves may serve as one or both of the aforementioned mating elements. Also, although the plurality of elements 1030 are shown on the second tube 1006, one skilled in the art will recognize that the plurality of elements 1030 may instead be disposed on the first tube, i.e., in an embodiment where the plurality of flaps are disposed on the second tube or on another tool/device used in conjunction with the surgical device. Similarly, the corresponding elements 1032 on the plurality of flaps 1004 may instead be disposed on one or more of the first tube 1002, the second tube 1006, or on another tool/device used in conjunction with the surgical device.

Fig. 11 shows a deployment mechanism in a deployed state. The deployment mechanism 1100 may be similar to the mechanism described above and may be implemented on a surgical tool having a first tube 1102, a plurality of flaps 1104, and a second tube 1106. As shown in the figure, the second tube 1106 may include a plurality of elements 1130 that each mechanically mate with a corresponding element 1132 on one of the plurality of flaps 1104, such that sliding the second tube 1006 in a first direction (as indicated by the arrow 1134) relative to the first tube 1102 causes the plurality of elements 1130 to engage the plurality of flaps 1104 and move each of the plurality of flaps 1104 from the undeployed state to the deployed state.

In use, starting from the undeployed state, as the second tube is moved towards the flaps, mating elements provided on each of the second tube and the flaps eventually engage. As the second tube continues towards the flaps, each of the mating elements begins to push on a corresponding element, thereby causing the flaps to deploy into their open configuration outside the transverse cross section of the tube(s).

Although the figures above depict relatively simple mechanical elements, in general the elements may be more complex. In particular, the mechanical elements may cause each flap to translate, swivel, rotate, or undergo other composite motion in order to extend beyond the end of the surgical apparatus to mechanically enforce separation between the tool and surrounding tissue.

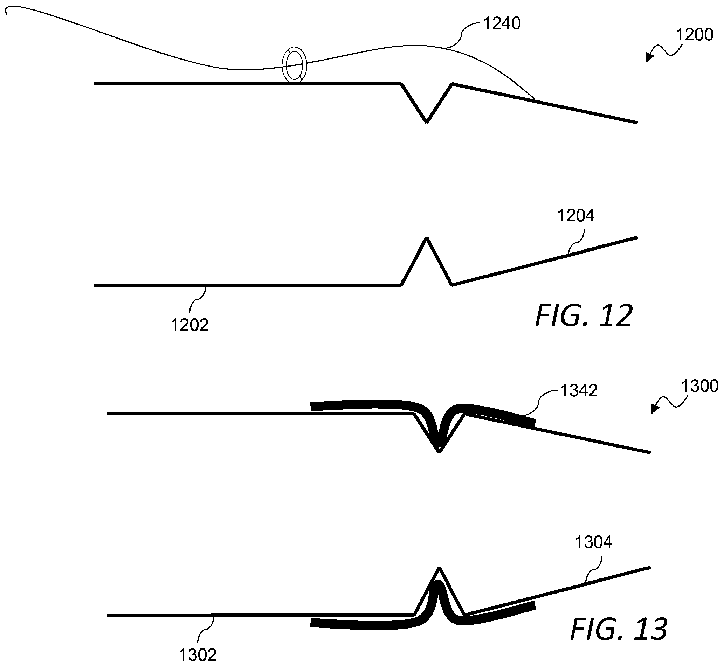

Fig. 12 shows a deployment mechanism in an undeployed state. The deployment mechanism 1200 may be similar to the mechanisms described above and may be implemented on a surgical tool having at least a first tube 1202 and a plurality of flaps 1204. As shown in the figure, the deployment mechanism 1200 may include one or more steering wires 1240. The one or more steering wires 1240 may be coupled to the plurality of flaps 1204 for controlling the plurality of flaps 1204, e.g., deployment of the plurality of flaps 1204. As stated above, the plurality of flaps 1204 may act as a protective shield in an embodiment, and thus the one or more steering wires 1240 may facilitate positional control of the protective shield during a surgical procedure so that the protective shield can be opened and closed as desired, and similarly so that the flaps can displace surrounding tissue as desired.

The one or more steering wires 1240 may be controlled by a controller as described herein, which can include any of the aforementioned controllers, e.g., the second controller described above for the supplemental tool. Thus, in an aspect, at least one steering wire 1240 couples the protective shield to the second controller on the proximal end of the first tube 1202 to facilitate positional control of the protective shield from the proximal end during a surgical procedure.

The one or more steering wires 1240 may be coupled to the surgical tool at various points. The coupling may be enabled by rings, loops, channels, grooves, or the like, which can advantageously maintain a close proximity between the one or more steering wires 1240 and the surgical tool, thereby avoiding unwanted or unanticipated contact with other anatomical structures. In some implementations, the one or more steering wires 1240 may each be coupled to an ancillary structure (e.g., a ring, or the like), and the ancillary structure may be coupled to the controller. This configuration may be advantageously used to synchronize and/or simplify the articulation of the plurality of flaps 1204.

Fig. 13 shows a surgical tool with a flexible coating. As shown in the figure, the surgical tool 1300 may include a tube 1302 (which may be either the first tube or the second tube described herein) having an associated flap 1304. A flexible coating 1342 may couple at least part of the flap 1304 to at least part of the tube 1302. The flap 1304 may further be coupled to the tube 1302 in other ways, e.g., as described above.

The flexible coating 1342 may advantageously be employed as a safety measure. For example, due to error or malfunction, a flap 1304 may break off from the tube 1302 during a surgical procedure. In this case, the broken flap may need to be retrieved from the patient to avoid further complications. However, such retrieval may be undesirable, insofar as it extends the length of the surgical procedure and puts the patient at risk for further injury. The presence of the flexible coating 1342 may mitigate the risk of the flap 1304 completely breaking off from the surgical tool 1300.

In some implementations, the flexible coating 1342 may include a plastic film, adhesive tape, a urethane, polyurethane, or polyimide coating, and the like.

The surgical tools including flaps as described above may further include one or more concentrically nested and curved tubes within the first tube operable to control a curvature of the first tube. The surgical tools including flaps as described above may also or include at least one of a magnet coupled to the device near the distal end, a control wire coupled between the proximal end and the distal end to control a positon of the distal end relative to the proximal end, and one or more balloons coupled to an exterior of the first tube and inflatable to move the first tube within a surgical cavity. The surgical tools described above may further include an endoscope positioned within the first tube.

Another implementation of a surgical apparatus for use in minimally invasive surgery and the like will now be described, where the surgical tool includes, inter alia, a cutting tool to cut through and allow access to the inside of the dura mater or the like.

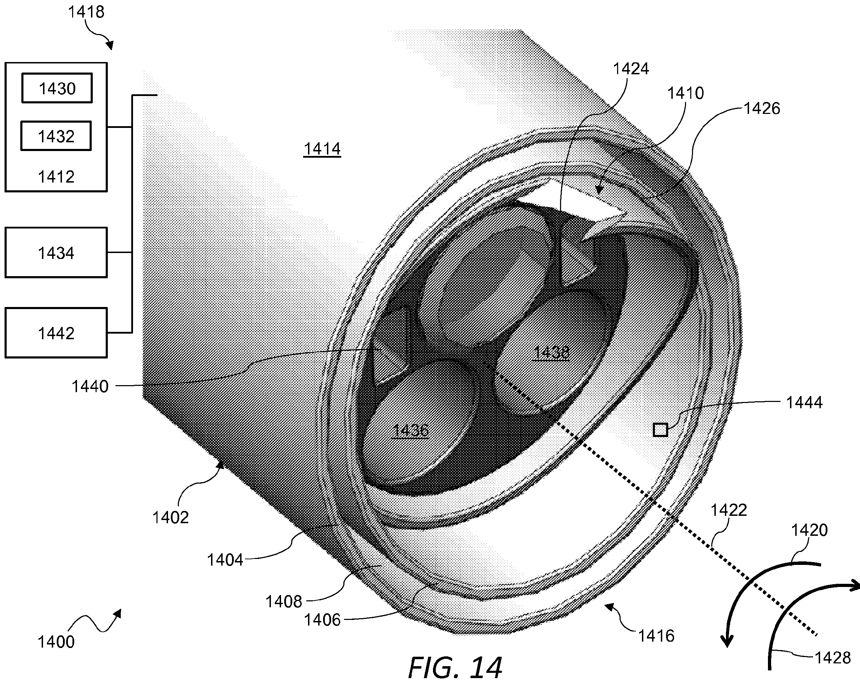

Fig. 14 shows a surgical apparatus. The surgical apparatus 1400 may allow for an endoscope or the like to access a sub-dural space during a spinal surgery. The surgical apparatus 1400 may include components that create suction to pull the dura mater away from sensitive neural tissue during surgery and concurrently secure the surgical apparatus 1400 against a wall of the tissue. This may allow a cutting tool or the like to cut or penetrate through the dura mater without damaging protected neural tissue.

The surgical apparatus 1400 may include a flexible endoscopic device 1402 (including a cannula, an endoscope, or any other similar component), an outer sheath 1404, an inner sheath 1406, a void 1408 between the outer sheath 1404 and the inner sheath 1406, a cutting tool 1410, and a controller 1412.

The flexible endoscopic device 1402 may include a body 1414 having a distal end 1416 for insertion into a surgical site and a proximal end 1418 for operator control (e.g., by the controller 1412 or a device in communication with the controller 1412, e.g., a computing device).

The outer sheath 1404 may be disposed on the distal end 1416 of the body 1414 of the flexible endoscopic device 1402. The outer sheath 1404 may also or instead extend the entire length of the flexible endoscopic device 1402 from the distal end 1416 to the proximal end 1418. The outer sheath 1404 may be movable or stationary with respect to one or more of the inner sheath 1406 and the cutting tool 1410, e.g., for one or both of rotational or translational motion.

The inner sheath 1406 may be disposed inside of and concentric with the outer sheath 1404 on the distal end 1416 of the body 1414 of the flexible endoscopic device 1402. The inner sheath 1406 may extend the entire length of the flexible endoscopic device 1402 from the distal end 1416 to the proximal end 1418, or the inner sheath 1406 or outer sheath 1404 or both may be a truncated cylindrical structure extending only far enough outside or within its neighboring sheath to provide a void space to apply suction as contemplated herein. The inner sheath 1406 may be movable or stationary with respect to one or more of the outer sheath 1404 and the cutting tool 1410, e.g., for one or both of rotational or translational motion.

The void 1408 may be disposed between the outer sheath 1404 and the inner sheath 1406 on the distal end 1416 of the body 1414 of the flexible endoscopic device 1402. The void 1408 may be in fluid connection with the proximal end 1418 of the body 1414. In this manner, the void 1408 may extend the entire length of the flexible endoscopic device 1402 from the distal end 1416 to the proximal end 1418. The arrangement of the void 1408 within the body may thus include a fluid communication configured such that a suction created at the proximal end 1418 of the body 1414 creates a reduced pressure in the void 1408 between the outer sheath 1404 and the inner sheath 1406 on the distal end 1416 of the body 1414 relative to an ambient pressure. In other words, suction created at the proximal end 1418 of the body 1414 (e.g., by a vacuum device, pump, or the like) may also cause suction at the distal end 1416 of the body at the void 1408. This may be advantageous, e.g., for fixing the body 1414 to a wall of a surgical site, and for drawing the surrounding tissue away from any sensitive anterior anatomical structures.

The cutting tool 1410 may be disposed at the distal end 1416 of the body 1414 of the flexible endoscopic device 1402. Specifically, as shown in the figure, in an aspect, the cutting tool 1410 is rotatably disposed inside of and concentric with the inner sheath 1406 where it is configured to rotate relative to the inner sheath 1406. Rotation of the cutting tool 1410 in a first direction (shown by the arrow 1420) about an axis 1422 may extend a sharp edge 1424 of the cutting tool 1410 beyond a leading edge 1426 of the inner sheath 1406. Rotation of the cutting tool 1410 in a second direction (shown by arrow 1428) about the axis 1422 may retract the sharp edge 1424 of the cutting tool 1410 such that it is fully sheathed by the leading edge 1426 of the inner sheath 1406. This may be provide a stowed position for the cutting tool 1410 where the sharp edge 1424 cannot cut tissue because it does not extend beyond the inner sheath 1406.

The cutting tool 1410 may also or instead be configured for translational movement with respect to one or more of the outer sheath 1404 and the inner sheath 1406, e.g., by axially moving the cutting tool 1410 to extend beyond the outer or inner sheath by a controllable amount.

The controller 1412 may be disposed at the proximal end 1418 of the body 1414 of the flexible endoscopic device 1402. The controller 1412 may include a first control 1430 to create suction at the proximal end 1418 of the body 1414 and a second control 1432 to rotate the cutting tool 1410 relative to the inner sheath 1406 (or otherwise control movement of one or more of the components of the flexible endoscopic device 1402).

The surgical apparatus 1400 may further include a vacuum source 1434 at the proximal end 1418 of the body 1414 that is coupled in fluid communication with the void 1408. The vacuum source 1434 may be configured to apply a variable vacuum force, e.g., under control of the controller 1412. In an aspect, the controller 1412 is configured to infer a vacuum sealed engagement of the distal end 1416 with a surface (e.g., a surface in a surgical site) based upon an amount of pressure created in the void 1408 by the vacuum source 1434. The controller 1412 may also or instead be configured to prevent operation of the cutting tool 1410 when no vacuum engagement of the distal end 1416 to a surface is detected. The controller 1412 may thus be in communication with one or more sensors (e.g., pressure sensors) disposed on the flexible endoscopic device 1402.

The body 1414 of the flexible endoscopic device 1402 may include one or more hollow cores therein, which form working channels for surgical tools to be included on the flexible endoscopic device 1402. For example, as shown in the figure, an implementation includes at least a first hollow core 1436 and a second hollow core 1438.

The first hollow core 1436 may include a surgical device. The surgical device may include without limitation a laser surgical tool, an electrosurgical radio frequency device, an imaging element, and so forth. The surgical device may be disposed at the distal end 1416 of the body 1414 and coupled to the controller 1412 at the proximal end 1418 through the first hollow core 1436.

The second hollow core 1438 in the body 1414 of the flexible endoscopic device 1402 may be parallel to the first hollow core 1436 for one or more supplemental tools. In an aspect, the flexible endoscopic device 1402 may include an imaging element disposed at the distal end 1416 (e.g., in the first hollow core 1436), where a supplemental tool includes a saline wash sourced through the second hollow core 1438 that is directed at the imaging element. The imaging element may include without limitation a digital camera at the distal end 1416, an optical fiber for optically transmitting an image at the distal end 1416 to the proximal end 1418, and so forth.

The flexible endoscopic device 1402 may also or instead include an illumination source 1440 at the distal end 1416. The illumination source 1440 may be coupled to a power source 1442 and a control source (e.g., the controller 1412) at the proximal end 1418.

A use case of the device discussed above will now be described. In use, with the sharp edge 1424 disposed (rotated) in the stowed position, the flexible endoscopic device 1402 may be navigated such that it is in direct contact with the dura mater. The suction between the outer sheath 1404 and the inner sheath 1406 may then be applied such that the dura mater is drawn toward the distal end 1416 of the flexible endoscopic device 1402. The flexible endoscopic device 1402 may then be pulled back such that the dura mater is pulled away from underlying neural elements (but not so far that the suction force is overcome and the dura mater is released). The cutting tool 1410 may then be rotated such that it cuts into the dura mater. Rotation may be continued until a hole is formed in the dura mater that is of adequate size to visualize the space therein and introduce any necessary tooling. In this manner, a substantially circular (or otherwise shaped) opening may be created, or a partial opening may be cut by the device. Tools may then be deployed to perform a procedure (e.g., tumor removal or the like) through the hole, or the flexible endoscopic device 1402 may be navigated inside of the dura. Tools may then be retracted into the flexible endoscopic device 1402 and suction may be released. The dura mater may then be sealed as required. Although suction or reduced pressure is described for securing a tool to the dura mater, it will be understood that a variety of other techniques may also or instead be used, including without limitation sutures, grasping mechanisms, tissue hooks, and so forth.

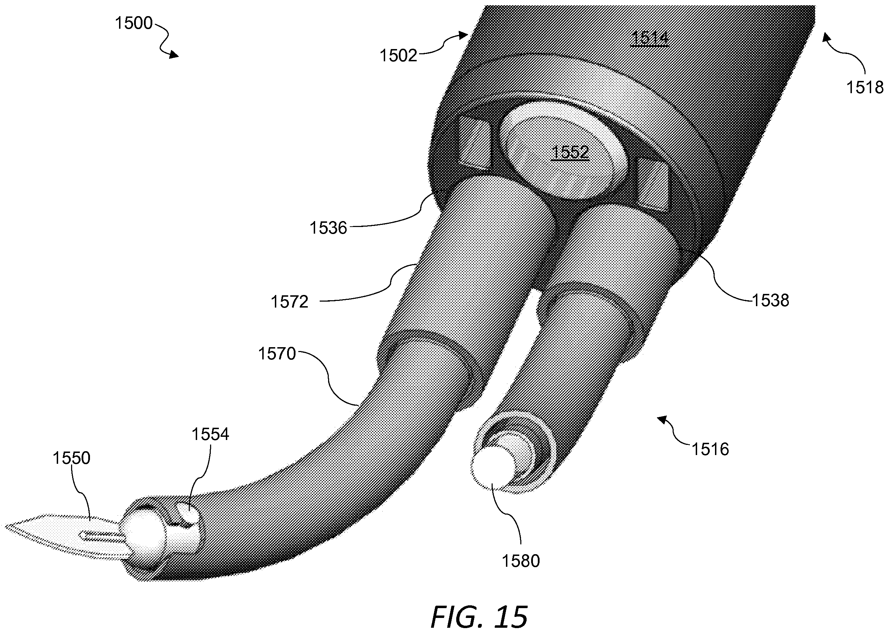

Fig. 15 shows a surgical apparatus with a surgical tool on a distal end thereof. The surgical apparatus 1500 may include a minimally invasive tool such as an endoscope. For example, similar to the apparatus discussed above, the surgical apparatus 1500 may include a flexible endoscopic device 1502, a body 1514 with a distal end 1516 and a proximal end 1518, and a plurality of hollow cores (e.g., a first hollow core 1536 and a second hollow core 1538). The surgical apparatus 1500 may further include a supplemental tool 1550 and a laser tool 1580 and an imaging element 1552.

As shown in the figure, in an aspect, the supplemental tool 1550 may include any suitable surgical tool such as a piercing tool, a hooking tool, a grasping tool, a cauterizer, a scissor, or any other tool that mighty be coupled to the body for use during a surgical procedure. In order to facilitate modular tool selection, the supplemental tool 1550 may be coupled to a sleeve 1570 or any other suitable attachment point with a locking mechanism 1554 that mechanically secures the supplemental tool 1550 to the surgical apparatus 1500 in a removable and replaceable manner. A variety of fixtures may be used for this purpose, but they should generally be sufficiently secure to prevent detachment during a procedure, and suitably shaped to avoid exposure of sharp points or cutting edges.

The supplemental tool 1550 may be disposed within or coupled to a steering mechanism such as a first sleeve 1570 and a second sleeve 1572 concentric with the first sleeve 1570. These sleeves 1570, 1572 may also provide a working channel (not shown) to electrically or mechanically couple the supplemental tool 1550 to a control end of the surgical apparatus 1500. The sleeves 1570, 1572 may be configured for steering the supplemental tool 1550 at the distal end 1516 of the surgical apparatus 1500. In one aspect, the sleeves 1570, 1572 form concentric tubes including a first tube (e.g., the first tube 1570) having an end with a predetermined radius of curvature and a predetermined stiffness, and a second tube (e.g., the second tube 1572) surrounding the first tube having a stiffness greater than the predetermined stiffness of the first tube and a radius of curvature greater than the predetermined radius of curvature of the first tube. In this manner, movement of at least one of the first tube and the second tube may enable steering of the supplemental tool 1550.

The supplemental tool 1550 may also include one or more stents, markers, fiducials or the like that can be used to visually locate the surgical apparatus 1500 or portions thereof. Techniques such as fluoroscopic guidance, intermittent x-ray imaging, or the like may be used to periodically verify the location of an instrument during a surgical procedure. Markers such as those described above can enhance the visibility of the surgical apparatus 1500, or a particular point on the surgical apparatus 1500 when viewed with a corresponding imaging device.

The imaging element 1552 may be any as described herein including a digital still camera, a video camera, or any other camera or similar device. One of the channels may also include any of the supplemental tools described herein. For example the second hollow core 1538 may contain a second laser surgical tool, supplemental lighting, graspers, or another mechanical or electrical device for use in a surgical procedure.

The surgical apparatus 1500 may further include a mechanical locking mechanism 1554 disposed at the distal end 1516 of the body 1514 of the endoscopic device 1502. As shown in the figure, the mechanical locking mechanism 1554 may be configured to removably and replaceably receive the supplemental tool 1550. In other words, the supplemental tool 1550 may be coupled to the mechanical locking mechanism 1554 in an embodiment. In another aspect, the mechanical locking mechanism 1554 may also or instead be configured to removably and replaceably receive the imaging element 1552.

The mechanical locking mechanism 1554 may mechanically fix the supplemental tool 1550 to the sleeve 1570 or otherwise to the body 1514 of the endoscopic device 1502. Specifically, the mechanical locking mechanism 1554 may couple the supplemental tool 1550 in a fixed engagement while in a first position, and the mechanical locking mechanism 1554 may decouple the supplemental tool 1550 while in a second position, where the supplemental tool 1550 may move independently from the sleeve 1570. In other words, there may be a rigid, semi-locking mechanical connection between a portion of the endoscopic device 1502 and the employed supplemental tool 1550. This configuration provides a platform for significant modularity, and may permit a high degree of versatility for the supplemental tool 1550. The mechanical locking mechanism 1554 may include locking or engagement features such as the protrusions shown in the figure, or it can include another locking feature, such as a magnetic force or any other implementation that results in the supplemental tool 1550 being firmly fixed to a portion of the endoscopic device 1502. Engagement and disengagement of the mechanical locking mechanism 1554 may be provided by a predetermined movement or series of movements of a component of the surgical apparatus 1500, e.g., by the combination of rotation of the supplemental tool 1550 in a specific direction followed by translation of the supplemental tool 1550 (i.e., a twist-lock configuration).

While the twist-lock configuration depicted in the figure is a useful mechanical connection of relatively simple design, it will be understood that a variety of twist lock configurations and other sufficiently mechanically secure removable/replaceable locking systems and the like are known in the art, and may be usefully adapted for use with the mechanical locking mechanism 1554 and supplemental tool 1550 contemplated herein. Further, active control systems using, e.g., remotely activated latches or electromagnetism may be used, for instance in circumstances where control over attachment and release is desired in a surgical context.

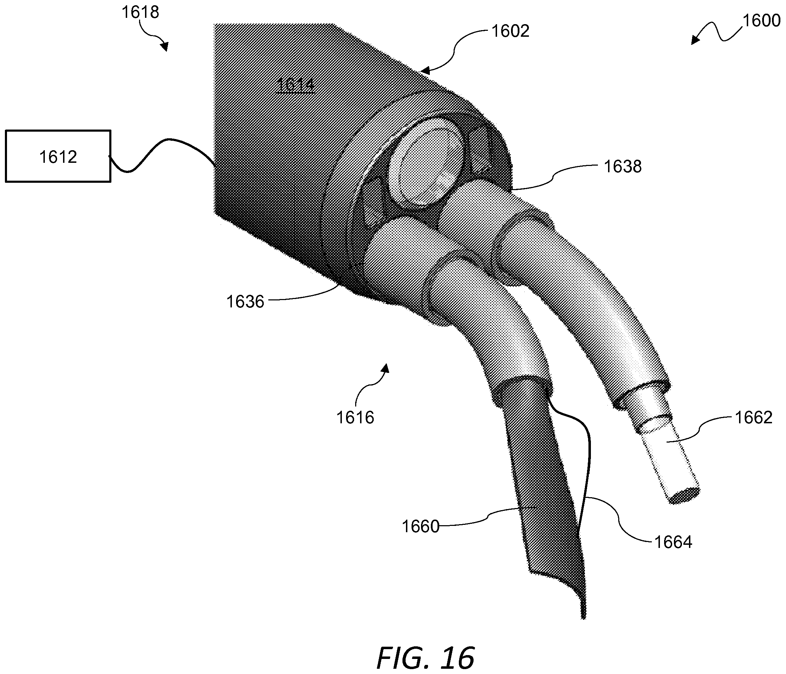

Fig. 16 shows a surgical apparatus with a protective shield on a distal end thereof. Similar to the apparatus discussed above, the surgical apparatus 1600 may include a flexible endoscopic device 1602, a body 1614 with a distal end 1616 and a proximal end 1618, and a plurality of hollow cores (e.g., a first hollow core 1636 and a second hollow core 1638). The surgical apparatus 1600 may further include a protective shield 1660 and a surgical laser 1662.

The plurality of hollow cores (e.g., a first hollow core 1636 and a second hollow core 1638) may include working channels of the surgical apparatus 1600, and as such they may include tools or the like for use in a surgical procedure (e.g., the protective shield 1660 and surgical laser 1662). One or more of the plurality of hollow cores may include concentric tubes as described herein. The tools contained within the hollow cores may thus be steerable and otherwise controllable.

The protective shield 1660 may be disposed at the distal end 1616 of the body 1614 as shown in the figure, e.g., in the first hollow core 1636. In an aspect, the protective shield 1660 is formed of a material selected to protect a surgical site from laser energy provided by a surgical laser operating at the distal end 1616 of the body 1614. The protective shield 1660 may thus include a laser shield, a laser/radiation absorber, or the like, e.g., fashioned of a biocompatible material that can be positioned over sensitive tissue in close proximity to laser treatment (e.g., by the surgical laser 1662), or more generally between the sensitive tissue and a laser source, to protect the sensitive tissue. The protective shield 1660 may usefully be fashioned of a laser absorbing material, or with a biocompatible laser absorbing finish, in order to more generally prevent scattering of laser energy around a surgical site.

The surgical laser 1662 may be disposed at the distal end 1616 of the body 1614 as shown in the figure, e.g., in the second hollow core 1638. The surgical laser 1662 may be any as discussed herein. Alternatively, in lieu of the surgical laser 1662, the second hollow core 1638 may instead include any of the surgical tools or supplemental tools described herein, where the protective shield 1660 protects a surgical site from these tools (or otherwise cooperates with these tools, e.g., guiding the tool or tissue within the surgical site).

The protective shield 1660 and the surgical laser 1662 may be independently movable with respect to one another, or they may be configured for synchronized movement. In an aspect, the protective shield 1660 and the surgical laser 1662 may be disposed in the same hollow core or in different working channels according to size and other physical constraints.

The surgical apparatus 1600 may further include at least one steering wire 1664 coupling the protective shield 1660 to a controller 1612 on the proximal end 1618 of the body 1614 to facilitate positional control of the protective shield 1660 from the proximal end 1618 during a surgical procedure. Positional control of the protective shield 1660 may also or instead be provided by other means, e.g., concentric tubes.

The surgical apparatus 1600 may further include a control mechanism (e.g., the controller 1612, which may include any of the controls discussed herein) to steer at least one of the protective shield 1660 and the surgical laser 1662 such that the protective shield 1660 protects areas around a surgical site being treated by the surgical laser 1662. In use, a protective shield 1660 could, for example, be maneuvered into a position to protect neural structures exiting the spinal canal as the inner radius of the area around the neural structure is increased via laser ablation or other means.

Although lasers have been usefully applied in a variety of surgical contexts as a tool for cutting or ablating tissue, there remains a need for a laser surgical tool to remove ligamentum flavum in minimally invasive surgical treatment for, e.g., spinal stenosis or similar conditions. Ligamentum flavum is a tough, rubbery connective tissue providing a portion of the ligamentous stability to the spinal column, and in a hypertrophied state this tissue forms a significant compressive pathology in degenerative spinal stenosis. The interaction of lasers and this biological tissue have not been thoroughly studied, and the problem is a challenging one raising design issues in disparate areas of lasers, optics, mechanics, and human anatomy/physiology. To date, no effective solution for laser ablation of ligamentum flavum has been demonstrated. By properly characterizing the various relevant heating and ablation mechanisms, and by devising a mechanical system capable of delivering targeted laser energy in constrained anatomical spaces, the applicant has devised a laser surgical system for treatment of spinal stenosis by removal of ligamentum flavum from within the spinal cavity. In the following description, the basic structure of a suitable laser system is described, followed by a number of characteristics and features of the resulting laser beam that affect performance in the context of tissue removal, and more particularly, ligamentum flavum removal.

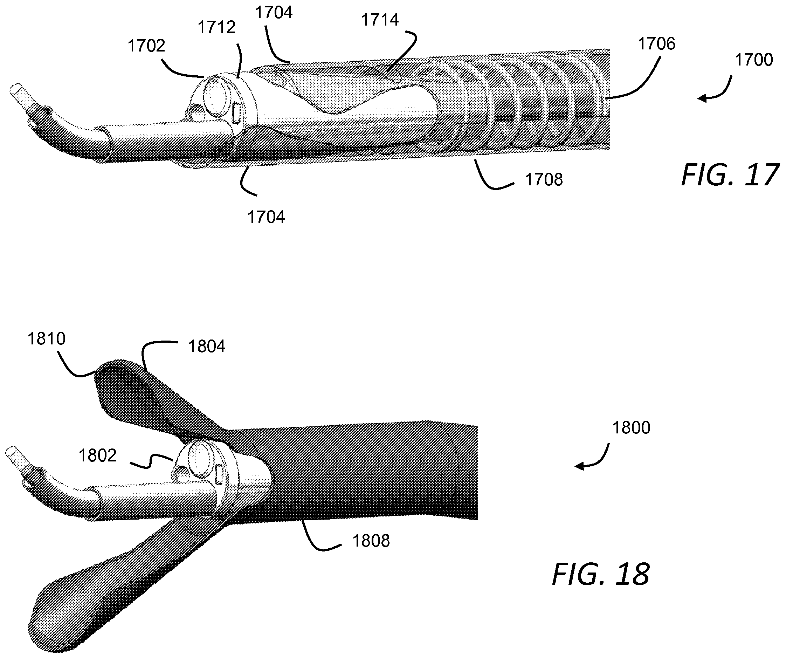

Fig. 17 shows an endoscopic body with a plurality of flaps in an undeployed state. The endoscopic body 1700 may in general include any of the endoscopic bodies or tools described herein, and may have a plurality of flaps 1702 or extendable flaps that can be deployed to provide physical separation from, and protection to, surrounding tissue during a surgical procedure as described above. In the embodiment of Fig. 17, the endoscopic tool 1704 is biased forward by a spring 1706 or the like inside an exterior sleeve 1708, and when the endoscopic tool 1704 is drawn backward into the sleeve (i.e., to the right in Fig. 17), this physical motion can apply a force to expand the plurality of flaps 1702 into a deployed position. More particularly as illustrated, a flange 1712 on the endoscopic tool 1704 may slide into contact with a protrusion 1714 on an interior surface of one of the plurality of flaps 1702 to rotate that one of the flaps 1702 away from a longitudinal axis of the endoscopic tool and out into the deployed position, as illustrated below in Fig. 18.

Fig. 18 shows an endoscopic body with a plurality of flaps in a deployed state. The endoscopic body 1800 may be the device shown in Fig. 17, with a plurality of flaps 1802 in a deployed state that provides physical separation from, and protection to, surrounding tissue during a surgical procedure. As depicted here, an endoscopic tool 1804 is pulled into an exterior sleeve 1808 (e.g., against a spring (not shown) or other biasing mechanism) thus physically displacing the plurality of flaps 1802 into a deployed position. It will be noted that, as described above, the plurality of flaps 1802 may have smooth, continuous edges 1810 in order to mitigate tearing or other damage to tissue during deployment and retraction.

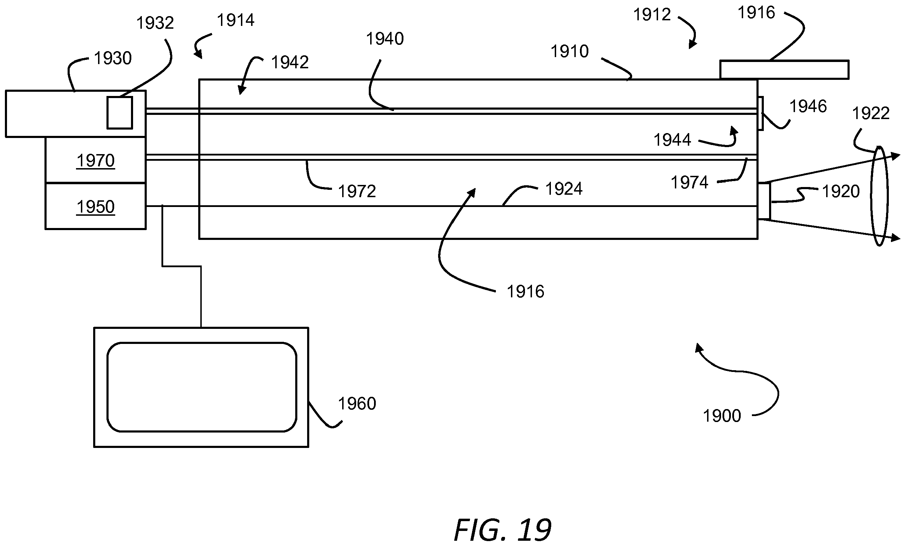

Fig. 19 shows an endoscopic device for removal of ligamentum flavum. In general, the endoscopic device 1900 may include a body 1910, a camera 1920, a laser 1930, a fiber optic 1940, and a controller 1950.

The body 1910 may in general be any of the endoscopic bodies described above for use in a minimally invasive surgical procedure. The body 1910 may, for example, be shaped and sized for insertion into a human spinal canal for use in a spinal cavity surgical procedure. The body may have a first end 1912 for insertion into a patient, a second end 1914 for controlling a surgical procedure, and a hollow core 1916 within the body 1910 between the first end 1912 and the second end 1914. The hollow core 1916 may include one or more channels or other passages or openings for functionally coupling a variety of tools on the first end 1912 where a procedure is performed to power, data, mechanical controls, and materials (e.g., saline) on the second end 1914 where a user controls a surgical procedure. The body 1910 may usefully incorporate a standoff at the first end 1912 including any structure or combination of structures suitable for enforcing a minimum separation distance and/or orientation between the first end 1912 (and the output of the optical fiber 1940) and a target surface. This may include a number of legs or the like, which may be fixed or retractable according to the intended use of the body 1910.

The camera 1920 may be any suitable still or video camera positioned on the first end 1912. The camera 1920 may have a field of view 1922 projecting from the first end 1912 where images of a surgical site can be obtained. The camera 1920 may be optically or electrically coupled with a coupling 1924 through the hollow core 1916 to the second end 1914 using any technique known in the art. The camera 1920 may be controlled with the controller 1950 and images from the camera 1920 may be presented on a display 1960 in order to provide visual feedback to a user during a procedure or to present output from the camera 1920 for any other reason.

The laser 1930 may be coupled to the second end 1914 of the body 1910 and may be operable to provide coherent light at a wavelength selected to remove ligamentum flavum from a surgical site. The wavelength may, for example, be an infrared wavelength selected to ablate ligamentum flavum through fracturing of extracellular matrix due to thermal heating of water preferentially over direct photodecomposition of extracellular matrix, e.g., where laser energy is absorbed by water, which subsequently heats and fractures the extracellular matrix. The peak absorption wavelength for water is around 1920 nanometers, although other higher and lower wavelengths in the infrared range also exhibit significant absorption and release of optical energy, and may also or instead be employed. For example the laser 1930 may include a Ho:YAG laser, a readily commercially available laser that outputs light at a wavelength of about 2080 nanometers. In this range where thermal heating of water is the primary mechanism for tissue destruction, the macroscopic mechanical and thermal damage to adjacent tissue can be extensive, requiring additional hardware to mitigate these fringe effects.

In another aspect, the wavelength may be an ultraviolet wavelength selected to ablate ligamentum flavum through direct photodecomposition of extracellular matrix preferentially over fracturing of extracellular matrix due to thermal heating of water, such as a laser that outputs light at a wavelength below 300 nanometers or a laser that outputs light below 400 nanometers. For example, the laser 1930 may include a frequency quadrupled Nd:YAG laser, a readily commercially available laser that outputs light at a wavelength of about 266 nanometers. This wavelength has been demonstrated to effectively remove tissue through direct photodecomposition-more specifically through decomposition of collagen bonds within ligamentum flavum tissue-while reducing fringe effects due to uncontained thermal heating or beam diffraction. As such, an ultraviolet laser may be particularly suitable for tissue removal in sensitive areas where some care and precision is required to avoid removing, e.g., sensitive neural tissue or the like. At the same time, it can be more difficult to transmit these wavelengths through conventional fiber optics, and remediation such as the use of high -OH (hydroxyl) fibers may be required.

The fiber optic 1940 may have a laser end 1942 coupled to the laser 1930 to direct an output of the laser 1930 through the hollow core 1916 to the first end 1912 of the body 1910 of the endoscopic device 1900 and out an output end 1944 toward a target within the field of view 1922 of the camera 1920.

In general, the output of the optical fiber 1940 may exhibit some diffraction of light exiting the output end 1944. This characteristic may be particularly problematic when using infrared lasers, where peripheral heating effects in the diffraction region may cause significant thermal heating without removing tissue, resulting in charring or other undesirable tissue damage. In order to mitigate these diffraction effects, the optical fiber 1940 may include an optical element 1946 that focuses a beam exiting the output end 1944 into a fluence spatial pattern having steep sides. This fluence spatial profile may be more specifically adapted to reduce heating of peripheral non-ablated tissue of a target surface exposed to the beam, i.e., tissue that is exposed to a portion of the beam with sufficient energy to heat and char the tissue without sufficient energy to decompose and remove the tissue. In one aspect, the optical element 1946 may optionally include a lens on the output end 1944 of the fiber optic 1940 that focuses the beam into a suitable diffraction-limited beam. In another aspect, the optical element may be the fiber optic 1940 itself, which may have a low Numerical Aperture selected to reduce a divergence of the beam at the output end 1944 of the fiber optic 1940. By way of non-limiting example, a Numerical Aperture of below 0.4 has been demonstrated to usefully reduce edge diffraction effects in an exiting beam. The properties of undesirable heating patterns, and spatial fluence profiles for mitigating same, are discussed in greater detail below.

In one aspect, the optical fiber 1940 may be selected to match an output wavelength of the fiber. For example a fiber with a low hydroxyl content not exceeding two parts per million may be usefully employed for infrared lasers, where the low hydroxyl content silica transmits infrared wavelengths more efficiently. Similarly, a fiber with high hydroxyl content not less than six hundred parts per million may be used to more efficiently transmit wavelengths in the ultraviolet range. High hydroxyl fiber may also exhibit brittleness, which may be mitigated with a hardened cladding or the like in order to facilitate bending for use in endoscopic applications.

The controller 1950 may include any suitable hardware and software coupled in a communicating relationship with other components of the endoscopic device 1900 and operable to control various aspects thereof. The controller 1950 may, for example, be coupled to the laser 1930 and programmed to deliver a sequence of pulses from the laser 1930 through the optical fiber 1940 and toward the target with sufficient fluence for bulk tissue removal of ligamentum flavum. A range of fluences may usefully achieve this objective. Depending on a number of other factors such as pulse length, fluence spatial profile, wavelength, and so forth, a fluence from about 35 Joules per centimeter squared to about 350 Joules per centimeter squared have been demonstrated to remove tissue in a single pulse of an Ho:YAG laser, with a substantially linear change in depth of tissue removal across this range. A multiple pulse technique with this laser yielded adequate tissue removal for use as a surgical tool with 30 Q-switched pulses at about 9 mJ/cm^2, although a lower limit for this technique was not investigated. For a frequency quadrupled Nd:YAG laser, adequate ablation was demonstrated between about 0.17 J/cm^2 and about 0.73 J/cm^2.

The endoscopic device 1900 may incorporate any of the supplemental tools described herein, including active tools (e.g., tools electromechanically manipulated from the control end of the body 1910) for imaging, tissue removal, laser steering and so forth, as well as passive tools for cutting, hooking, and so forth. For example, the endoscopic device 1900 may include a material supply 1970 such as a saline source coupled to a saline output 1974 at the first end 1912 of the body through a material delivery duct 1972 or the like. Where a saline wash is provided from the material supply 1970 through the material delivery duct 1972 (e.g., through operation of a manual or electromechanical pump), the saline wash may usefully augment a surgical procedure in a number of ways. For example, the saline wash may be directed toward a camera or light source in order to clean or clear debris that might obstruct optical functions. The saline wash may also or instead be directed toward a surgical site for similar reasons, or to provide cooling during a laser surgical procedure. For example, where heating from a laser is poorly contained, there may be significant thermal damage to tissue surrounding a target without removal of corresponding tissue. In order to mitigate these types of edge effects during laser tissue removal, a saline wash may be periodically applied in order to cool tissue around the edge of a fluence profile for a laser.

In another aspect, the entire spinal canal may be filled and slightly pressurized with saline in order to effectively depress the dura mater and provide an expanded physical space for movement and visibility. In another useful example of fluid delivery, the material supply 1970 may contain an epoxy, surgical glue, or other liquid to replace degenerated or diseased disc material or bone. This may include materials that can be cured into a biosimilar structure to match the mechanical and other properties of surrounding tissue as necessary or desirable for proper physiological function, as well as any therapeutic materials, growth factors, or other compounds to support regrowth and repair of existing tissue, or provide a scaffolding for same. Where curing is desired for such a material, an ultraviolet light source, heating element, or other active device may be deployed on the first end 1912 of the body 1910 to facilitate this curing process under control of the controller 1950.

The endoscopic device 1900 may optionally include a Q-switch 1932 operable to produce a pulsed laser output from the laser 1930. In general, the Q-switch may be any variable optical attenuator such as an acousto-optic attenuator or the like positioned within an optical resonator of the laser 1930 to control energy stored within the laser gain medium and release the stored energy in short pulses. A variety of attenuators are known in the art and may be adapted to control operation of the laser 1930 as contemplated herein, more specifically by permitting shorter pulse lengths with useful surgical properties. The controller 1950 may be coupled to the laser 1930 and the Q-switch 1932 and programmed to deliver a sequence of Q-switched pulses from the laser 1930 through the optical fiber 1940 toward a target with a sufficient fluence for bulk tissue removal of ligamentum flavum and with a pulse length that is sufficiently short to prevent regions of reduced fluence in a beam exiting from the output end from causing non-ablative heating damage to regions of tissue around a periphery of the target.

For example, without a Q-switch, a commercially available Ho:YAG laser might be operated with a pulse length of about 140 microseconds full width half maximum (FWHM). Using the Q-switch 1932, the controller 1950 may be configured to operate the same laser at a significantly shorter pulse length such as a pulse length of about 260 nanoseconds FWHM. Similarly, with a Q-switch the controller 1950 may drive the laser 1930 with a pulse length of not more than 20 nanoseconds FWHM, not more than 5 nanoseconds FWHM, not more than 1 nanosecond FWHM, or not more than 1 picosecond FWHM.

By using shorter pulse lengths in this manner, the treated area has less time to increase in temperature due to thermal heating, and as such the requirements for thermal control can be relaxed. This may, for example, relieve the need for frequent saline rinsing to reduce temperature between exposures, or reduce the need for focusing lenses that might otherwise be necessary to produce a diffraction-limited beam. At still shorter pulse lengths, e.g., in the femtosecond range at 1 picosecond or less, pulses can achieve very high ablation efficiency with virtually no artifacts of thermal heating such as stress, deformation, charring, and so forth. Lasers operating in this range can provide significantly sharper and cleaner edges around a target site, and further reduce any need for supplemental thermal or optical management.

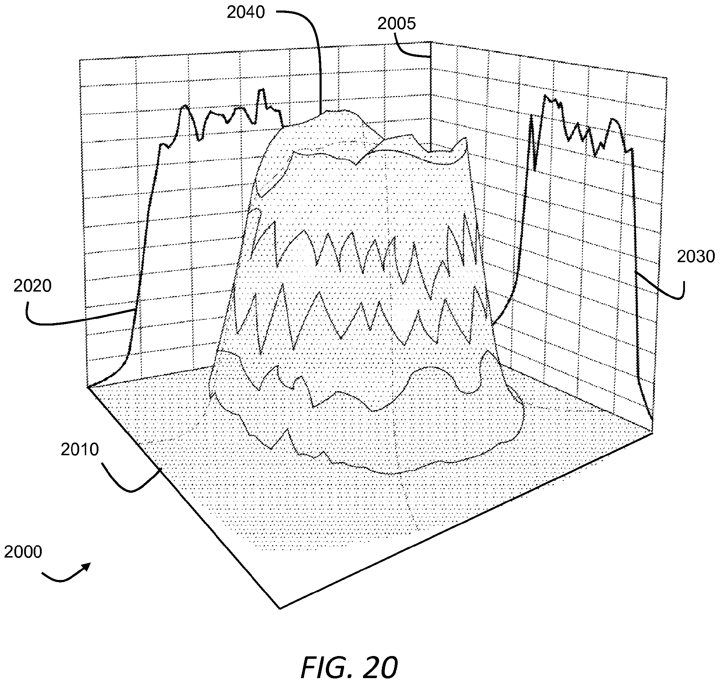

Fig. 20 shows a fluence spatial pattern for a laser on a target surface. In general, the fluence spatial pattern characterizes the intensity (e.g., in Joules per centimeter squared (J/cm^2)) in a beam as height on a z-axis 2005 according to location in a horizontal plane 2010 that the beam intersects, which may approximate a target surface for a surgical procedure. It will be noted that the three-dimensional surface 2040 bounded by this fluence spatial pattern 2000 forms a substantially top-hat pattern or cylinder characterized by steep roll-off at a periphery of the beam and a relatively uniform energy within the beam region. This can also be seen in a first cross section 2020 and a second cross section 2030 of the fluence in a plane passing through a center of the beam region. The steepness of this roll-off can be characterized in a variety of ways. For example, the steepness of the edges of the top-hat profile may be measured as a normalized or average difference between areas of 10% of peak and 90% of peak. An adequate profile for a Ho:YAG laser using this metric, for example, may be between 0.9 and 1.0 (perfectly vertical). The fluence spatial pattern may be similarly characterized according to side lobes, which are preferably at least 30 dB below the peak value within the beam region. It will be appreciated that the actual fluence spatial pattern may vary according to the wavelength of light used and the fiber that the light is transmitted through. Particularly in applications with infrared lasers, where thermal heating can be harmful to peripheral tissue, or more generally where poor thermal containment of the ablated region results in tissue damage to intact tissue around a target area, lenses or other optical elements may be usefully incorporated to improve the fluence spatial pattern 2000 for more uniform ablation results.

The apparatuses relating to the cutting tool, the locking mechanism, and the protective shield discussed above may be outfitted, supplemented, or may have elements replaced with any of the other features and tools otherwise described herein and/or may be combined with one another or other surgical tool features described herein for performing surgical operations or steering an endoscopic within a spinal canal or similarly constrained space. For example, the surgical apparatus may include one or more concentrically nested and curved tubes within the body of the endoscopic device. The concentrically nested and curved tubes may be operable to control a curvature of tubes of a surgical tool (or other device) disposed in a hollow core. The surgical apparatus may also or instead include at least one of a magnet coupled to the device near the distal end, a control wire coupled between the proximal end and the distal end to control a positon of the distal end relative to the proximal end, one or more balloons coupled to an exterior of the body and inflatable to move the body within a surgical cavity, and so forth. The surgical apparatus may also or instead include an endoscope positioned within device, e.g., in one of the hollow cores therein, or the body may otherwise include an endoscope.

The above systems, devices, methods, processes, and the like described herein may be used in endoscopic surgery within the spinal canal. An envisioned use includes spinal decompression by ablation of ligamentum flavum, wherein the endoscope is introduced through the sacral hiatus. With the addition of the tools disclosed herein, surgeries of increased complexity can be performed. A laser shield could, for example, be used to protect neural structures exiting the spinal canal as the inner radius of the area around the neural structure is increased via laser ablation or other means. The surgical tools may also or instead enable endoscopic access inside of the dura mater, allowing for tumor removal when the tumor is in direct contact with the spinal cord or other neural structures.

The above systems, devices, methods, processes, and the like may be realized in hardware, software, or any combination of these suitable for a particular application. The hardware may include a general-purpose computer and/or dedicated computing device. This includes realization in one or more microprocessors, microcontrollers, embedded microcontrollers, programmable digital signal processors or other programmable devices or processing circuitry, along with internal and/or external memory. This may also, or instead, include one or more application specific integrated circuits, programmable gate arrays, programmable array logic components, or any other device or devices that may be configured to process electronic signals. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software. In another aspect, the methods may be embodied in systems that perform the steps thereof, and may be distributed across devices in a number of ways. At the same time, processing may be distributed across devices such as the various systems described above, or all of the functionality may be integrated into a dedicated, standalone device or other hardware. In another aspect, means for performing the steps associated with the processes described above may include any of the hardware and/or software described above. All such permutations and combinations are intended to fall within the scope of the present disclosure.

Embodiments disclosed herein may include computer program products comprising computer-executable code or computer-usable code that, when executing on one or more computing devices, performs any and/or all of the steps thereof. The code may be stored in a non-transitory fashion in a computer memory, which may be a memory from which the program executes (such as random access memory associated with a processor), or a storage device such as a disk drive, flash memory or any other optical, electromagnetic, magnetic, infrared or other device or combination of devices. In another aspect, any of the systems and methods described above may be embodied in any suitable transmission or propagation medium carrying computer-executable code and/or any inputs or outputs from same.

## Claims

1. A device (100, 200, 300, 400, 500) comprising:
a surgical tool (108) having a body (110, 210, 310, 410, 510), a control end (114), and a distal end (116, 316, 416), the body shaped and sized for insertion past a sacral hiatus and into a spinal canal of a patient, the control end positioned outside the patient when the surgical tool is positioned for use, and the distal end positioned inside the spinal canal when the surgical tool is positioned for use;
a compound curve (112, 212) in the body of the surgical tool, the compound curve including at least a first bend (118, 218) with a predetermined stiffness positioned to provide a point of contact between the surgical tool and a supporting tissue region of the spinal canal when a distal tip (117, 317) of the distal end is positioned for a procedure on a surgical target within the spinal canal, thereby providing mechanical support for a position of the distal tip, and the compound curve including a second bend (120, 220) with a predetermined stiffness positioned to direct the distal tip toward the surgical target; and
one or more balloons (540) coupled to an exterior of the body and inflatable to move the body within the spinal canal, which, when inflated, are operable to distribute a contact force of the body of the device at the point of contact between the surgical tool and the supporting tissue region.

2. The device of claim 1 wherein the predetermined stiffness of the first bend is selected to deform to a native curvature of the spinal canal in order to retain adequate mating contact between the body and a wall of the spinal canal in a region between a first apex of the first bend and a second apex of the second bend to retain the distal end in a fixed position relative to the surgical target.

3. The device of claim 2 wherein the predetermined stiffness of the first bend retains the second apex in contact with the wall of the spinal canal.

4. The device of any preceding claim wherein the second bend directs the distal end toward the surgical target when the surgical tool is placed for use.

5. The device of any preceding claim wherein the surgical tool is an endoscope or a cannula for an endoscope.

6. The device of any preceding claim further comprising at least one of a magnet (434) coupled to the body near the distal end, a control wire (332) coupled between the control end and the distal end to control a position of the distal end relative to the body, and one or more concentrically nested and curved tubes within the body operable to control a curvature of the second bend.

7. The device of any preceding claim wherein the body has an outside diameter not greater than 3 mm.

8. The device of any preceding claim further comprising a surgical laser and an optical fiber positioned to transmit optical energy from the surgical laser through the body to the distal tip.

9. The device of any preceding claim further comprising an electrosurgical radio frequency device coupled to the distal end and operable for radio frequency ablation of the surgical target.

10. The device of claim 1 further comprising a mechanical locking mechanism at the distal end configured to removably and replaceably receive at least one of a piercing tool, a cutting tool, a hooking tool, and a grasping tool coupled to the mechanical locking mechanism.

11. The device of claim 1 further comprising a protective shield at the distal end formed of a material selected to protect a surgical site from laser energy provided by a surgical laser operating at the distal end.

12. The device of claim 1 further comprising:
a camera on the distal end, the camera having a field of view projecting from the distal end and the camera coupled through the body to a display on the control end;
a laser coupled to the distal end and operable to provide coherent light at an infrared wavelength selected to ablate ligamentum flavum through fracturing of extracellular matrix due to thermal heating of water preferentially over direct photodecomposition of extracellular matrix;
a fiber optic having a laser end coupled to the laser to direct an output of the laser through the body to the distal end of the device and out an output end toward a target within the field of view of the camera, the fiber optic including an optical element that focuses a beam exiting the output end into a fluence spatial pattern having steep sides to reduce heating of peripheral nonablated tissue of a target surface exposed to the beam; and
a controller coupled to the laser and programmed to deliver a sequence of pulses from the laser through the fiber optic toward the target with a sufficient fluence for bulk tissue removal of ligamentum flavum.

13. The device of claim 12 wherein the optical element includes one or more of a lens on the output end of the fiber optic that focuses the beam into a diffraction limited beam, and a low Numerical Aperture of the fiber optic selected to reduce divergence of the beam at the output end of the fiber optic.

14. The device of claim 12 wherein the fluence spatial pattern is at least one of a substantially top-hat pattern and a pattern that has side lobes at least 30 dB below a peak of the fluence spatial pattern.

15. The device of claim 12 further comprising a saline source coupled to a saline output at the distal end of the body.

## Patentansprüche

1. Vorrichtung (100, 200, 300, 400, 500), umfassend:
ein chirurgisches Instrument (108), welches einen Körper (110, 210, 310, 410, 510), ein Steuerende (114) und ein distales Ende (116, 316, 416) aufweist, wobei der Körper zum Einführen über einen Hiatus sacralis hinaus und in einen spinalen Kanal eines Patienten geformt und dimensioniert ist, wobei das Steuerende außerhalb des Patienten angeordnet ist, wenn das chirurgische Instrument zur Verwendung positioniert ist, und wobei das distale Ende innerhalb des spinalen Kanals angeordnet ist, wenn das chirurgische Instrument zur Verwendung positioniert ist;
ein Pufferelement (112, 212) in dem Körper des chirurgischen Instruments, wobei das Pufferelement mindestens eine erste Biegung (118, 218) mit einer vorbestimmten Steifigkeit umfasst, welche positioniert ist, um einen Kontaktpunkt zwischen dem chirurgischen Instrument und einem unterstützenden Gewebebereich des spinalen Kanals bereitzustellen, wenn eine distale Spitze (117, 317) des distalen Endes positioniert ist, um einen Eingriff auf einem chirurgischen Ziel innerhalb des spinalen Kanals auszuführen, wodurch eine mechanische Stützung für eine Position der distalen Spitze bereitgestellt wird, und das Pufferelement eine zweite Biegung (120, 220) umfasst, welche eine vorbestimmte Steifigkeit aufweist, welche positioniert ist, um die distale Spitze zum chirurgischen Ziel zu leiten; und
einen oder mehrere Ballons (540), welche mit einer Außenseite des Körpers gekoppelt und aufblasbar sind, um den Körper innerhalb des spinalen Kanals zu bewegen, welche, wenn aufgeblasen, wirksam sind, um eine Kontaktkraft des Körpers der Vorrichtung an dem Kontaktpunkt zwischen dem chirurgischen Instrument und dem unterstützenden Gewebebereich zu verteilen.

2. Vorrichtung nach Anspruch 1, wobei die vorbestimmte Steifigkeit der ersten Biegung ausgewählt wird, um sich zu einer nativen Krümmung des spinalen Kanals zu verformen, um einen geeigneten Kopplungskontakt zwischen dem Körper und einer Wand des spinalen Kanals in einem Bereich zwischen einem ersten Apex der ersten Biegung und einem zweiten Apex der zweiten Biegung aufrechtzuerhalten, um das distale Ende in einer festen Position relativ zum chirurgischen Ziel zu halten.

3. Vorrichtung nach Anspruch 2, wobei die vorbestimmte Steifigkeit der ersten Biegung den zweiten Apex in Kontakt mit der Wand des spinalen Kanals hält.

4. Vorrichtung nach einem der vorhergehenden Ansprüche, wobei die zweite Biegung das distale Ende zum chirurgischen Ziel leitet, wenn das chirurgische Instrument zur Verwendung positioniert wird.

5. Vorrichtung nach einem der vorhergehenden Ansprüche, wobei das chirurgische Instrument ein Endoskop oder eine Kanüle für ein Endoskop ist.

6. Vorrichtung nach einem der vorhergehenden Ansprüche, ferner umfassend mindestens eines von einem Magneten (434), welcher mit dem Körper in der Nähe des distalen Endes gekoppelt ist, einem Steuerdraht (332), welcher zwischen dem Steuerende und dem distalen Ende gekoppelt ist, um eine Position des distalen Endes relativ zum Körper zu steuern, und einem oder mehreren konzentrisch verschachtelten und gekrümmten Röhren innerhalb des Körpers, welche wirksam sind, um eine Krümmung der zweiten Biegung zu steuern.

7. Vorrichtung nach einem der vorhergehenden Ansprüche, wobei der Körper einen Außendurchmesser aufweist, welcher nicht größer als 3 mm ist.

8. Vorrichtung nach einem der vorhergehenden Ansprüche, ferner umfassend einen chirurgischen Laser und eine optische Faser, welche angeordnet ist, um optische Energie von dem chirurgischen Laser durch den Körper bis zur distalen Spitze zu übertragen.

9. Vorrichtung nach einem der vorhergehenden Ansprüche, ferner umfassend eine elektro-chirurgische Funkfrequenzvorrichtung, welche mit dem distalen Ende gekoppelt ist und wirksam ist, um eine Funkfrequenzablation des chirurgischen Ziels auszuführen.

10. Vorrichtung nach Anspruch 1, ferner umfassend einen mechanischen Verriegelungsmechanismus an dem distalen Ende, welcher konfiguriert ist um mindestens eines von einem Stechwerkzeug, einem Schneidwerkzeug, einem Kupplungswerkzeug und einem Greifwerkzeug lösbar und austauschbar aufzunehmen, welches mit dem mechanischen Verriegelungsmechanismus gekoppelt ist.

11. Vorrichtung nach Anspruch 1, ferner umfassend einen Schutzschild am distalen Ende, welcher aus einem Material besteht, welches ausgewählt wird, um eine Eingriffsstelle gegen Laserenergie zu schützen, welche durch einen chirurgischen Laser bereitgestellt wird, welcher an dem distalen Ende operiert.

12. Vorrichtung nach Anspruch 1, ferner umfassend:
eine Kamera an dem distalen Ende, wobei die Kamera ein Sichtfeld aufweist, welches von dem distalen Ende vorsteht und wobei die Kamera durch den Körper mit einer Anzeige auf dem Steuerende gekoppelt ist;
einen Laser, welcher mit dem distalen Ende gekoppelt ist und wirksam ist, um kohärentes Licht mit einer infraroten Wellenlänge bereitzustellen, welche ausgewählt wird, um ligamentum flavum durch Frakturieren der extrazellularen Matrix durch thermische Erwärmung von Wasser, bevorzugt durch eine direkte Photozersetzung der extrazellularen Matrix;
eine optische Faser, welche ein Laserende aufweist, welches mit dem Laser gekoppelt ist, um eine Laserausgabe durch den Körper zum distalen Ende der Vorrichtung zu leiten und um diese aus einem Ausgabeende zu einem Ziel innerhalb des Sichtfelds der Kamera zu leiten, wobei die optische Faser ein optisches Element umfasst, welches einen Strahl fokussiert, der aus dem Ausgabeende austritt und in ein räumliches Flussdichtemuster, welches steile Seiten aufweist, um die Erwärmung des peripheren nicht-ablatierten Gewebes einer Zielfläche zu reduzieren, welche dem Strahl ausgesetzt ist; und
eine Steuerung, welche mit dem Laser gekoppelt ist und programmiert ist, um eine Sequenz von Impulsen von dem Laser durch die optische Faser zum Ziel mit einer ausreichenden Flussdichte auszugeben, zur massiven Gewebeentfernung von ligamentum flavum.

13. Vorrichtung nach Anspruch 12, wobei das optische Element eine oder mehrere Linsen an dem Ausgabeende der optischen Faser umfasst, welche den Strahl in einen beugungsarmen Strahl fokussieren, und wobei eine numerische Apertur der optischen Faser ausgewählt ist, um die Divergenz des Strahls an dem Ausgabeende der optischen Faser zu reduzieren.

14. Vorrichtung nach Anspruch 12, wobei das räumliche Flussdichtemuster mindestens eines von einem im Wesentlichen Top-Hat-Muster und einem Muster, welches seitliche Lobe aufweist, welche mindestens 30 dB unterhalb einer Spitze des räumlichen Flussdichtenmusters liegt.

15. Vorrichtung nach Anspruch 12, ferner umfassend eine Salzquelle, die mit einer Salzausgabe an dem distalen Ende des Körpers gekoppelt ist.

## Revendications

1. Dispositif (100, 200, 300, 400, 500) comprenant :
un instrument chirurgical (108) qui comporte un corps (110, 210, 310, 410, 510), une extrémité de commande (114) et une extrémité distale (116, 316, 416), le corps étant conformé et dimensionné pour son insertion au-delà d'une échancrure sacrée et à l'intérieur d'un canal rachidien d'un patient, l'extrémité de commande étant positionnée à l'extérieur du patient lorsque l'instrument chirurgical est positionné pour son utilisation, et l'extrémité distale étant positionnée à l'intérieur du canal rachidien lorsque l'instrument chirurgical est positionné pour son utilisation ;
une courbe composée (112, 212) dans le corps de l'instrument chirurgical, la courbe composée incluant au moins une première partie courbe (118, 218) qui présente une raideur prédéterminée et qui est positionnée pour assurer un point de contact entre l'instrument chirurgical et une région de tissu de support du canal rachidien lorsqu'une pointe distale (117, 317) de l'extrémité distale est positionnée pour une procédure sur une cible chirurgicale à l'intérieur du canal rachidien, d'où ainsi la réalisation d'un support mécanique pour une position de la pointe distale, et la courbe composée incluant une seconde partie courbe (120, 220) qui présente une raideur prédéterminée et qui est positionnée pour diriger la pointe distale en direction de la cible chirurgicale ; et
un ou plusieurs ballonnet(s) (540) qui est/sont couplé(s) sur un extérieur du corps et qui peut/peuvent être gonflé(s) pour déplacer le corps à l'intérieur du canal rachidien, lesquels ballonnets, lorsqu'ils sont gonflés, sont opérationnels pour imprimer une force de contact du corps du dispositif au niveau du point de contact entre l'instrument chirurgical et la région de tissu de support.

2. Dispositif selon la revendication 1, dans lequel la raideur prédéterminée de la première partie courbe est sélectionnée pour réaliser une déformation selon une courbure native du canal rachidien afin de maintenir un contact d'appariement adéquat entre le corps et une paroi du canal rachidien dans une région entre un premier apex de la première partie courbe et un second apex de la seconde partie courbe pour maintenir l'extrémité distale dans une position fixe par rapport à la cible chirurgicale.

3. Dispositif selon la revendication 2, dans lequel la raideur prédéterminée de la première partie courbe maintient le second apex en contact avec la paroi du canal rachidien.

4. Dispositif selon l'une quelconque des revendications qui précèdent, dans lequel la seconde partie courbe dirige l'extrémité distale en direction de la cible chirurgicale lorsque l'instrument chirurgical est positionné pour son utilisation.

5. Dispositif selon l'une quelconque des revendications qui précèdent, dans lequel l'instrument chirurgical est un endoscope ou une canule pour un endoscope.

6. Dispositif selon l'une quelconque des revendications qui précèdent, comprenant en outre au moins un parmi un aimant (434) qui est couplé au corps à proximité de l'extrémité distale, un fil de commande (332) qui est couplé entre l'extrémité de commande et l'extrémité distale pour commander une position de l'extrémité distale par rapport au corps, et un ou plusieurs tube(s) emboîtés et incurvés de façon concentrique à l'intérieur du corps, lesquels sont opérationnels pour commander une courbure de la seconde partie courbe.

7. Dispositif selon l'une quelconque des revendications qui précèdent, dans lequel le corps présente un diamètre externe qui n'est pas supérieur à 3 mm.

8. Dispositif selon l'une quelconque des revendications qui précèdent, comprenant en outre un laser chirurgical et une fibre optique qui est positionnée pour transmettre l'énergie optique en provenance du laser chirurgical à travers le corps jusqu'à la pointe distale.

9. Dispositif selon l'une quelconque des revendications qui précèdent, comprenant en outre un dispositif radiofréquence électrochirurgical qui est couplé à l'extrémité distale et qui est opérationnel pour l'ablation radiofréquence de la cible chirurgicale.

10. Dispositif selon la revendication 1, comprenant en outre un mécanisme de blocage mécanique au niveau de l'extrémité distale qui est configuré pour recevoir de manière amovible et de façon interchangeable au moins un parmi un instrument de poinçonnage, un instrument de coupe, un instrument de crochetage et un instrument de préhension qui est couplé au mécanisme de blocage mécanique.

11. Dispositif selon la revendication 1, comprenant en outre une gaine de protection au niveau de l'extrémité distale qui est formée à partir d'un matériau qui est sélectionné pour protéger un site chirurgical vis-à-vis de l'énergie laser qui est fournie par un laser chirurgical qui opère au niveau de l'extrémité distale.

12. Dispositif selon la revendication 1, comprenant en outre :
une caméra sur l'extrémité distale, la caméra présentant un champ de visualisation en projection depuis l'extrémité distale et la caméra étant couplée par l'intermédiaire du corps à un affichage sur l'extrémité de commande ;
un laser qui est couplé à l'extrémité distale et qui est opérationnel pour fournir une lumière cohérente à une longueur d'onde des infrarouges qui est sélectionnée pour réaliser l'ablation du ligament jaune par l'intermédiaire de la fracturation de la matrice extracellulaire du fait du chauffage thermique d'eau, de façon préférentielle à la photodécomposition directe de la matrice extracellulaire ;
une fibre optique qui comporte une extrémité laser qui est couplée au laser pour diriger une sortie du laser au travers du corps jusqu'à l'extrémité distale du dispositif et hors d'une extrémité de sortie en direction d'une cible à l'intérieur du champ de visualisation de la caméra, la fibre optique incluant un élément optique qui focalise un faisceau qui sort de l'extrémité de sortie selon un motif spatial de fluence qui comporte des côtés raides pour réduire le chauffage du tissu périphérique non soumis à ablation d'une surface cible qui est exposée au faisceau ; et
un contrôleur qui est couplé au laser et qui est programmé pour délivrer une séquence d'impulsions depuis le laser au travers de la fibre optique en direction de la cible selon une fluence suffisante pour l'extraction d'une grande partie du tissu du ligament jaune.

13. Dispositif selon la revendication 12, dans lequel l'élément optique inclut une ou plusieurs lentilles sur l'extrémité de sortie de la fibre optique qui focalise le faisceau en un faisceau à diffraction limitée, et une ouverture numérique faible de la fibre optique qui est sélectionnée pour réduire la divergence du faisceau au niveau de l'extrémité de sortie de la fibre optique.

14. Dispositif selon la revendication 12, dans lequel le motif spatial de fluence est au moins un parmi un motif sensiblement en forme de chapeau haut de forme et un motif qui comporte des lobes latéraux à au moins 30 dB en-deçà d'une crête du motif spatial de fluence.

15. Dispositif selon la revendication 12, comprenant en outre une source de solution saline qui est couplée à une sortie de solution saline au niveau de l'extrémité distale du corps.
